# EUROPEAN PATENT APPLICATION

(11) **EP 1 742 060 A1**
(43) Date of publication of application: **10.01.2007**
(21) Application number: 05001341.6
(22) Date of filing: 24.01.2005
(51) Int. Cl.: G01N 33/574

(54) **TIF1-beta peptides and nucleic acids for diagnosis and therapy of cancer and colorectal cancerous disorders.**

(71) Applicant: DIGILAB BioVisioN GmbH, 30625 Hannover (DE)
(72) Inventor: Tammen, Harald, 30625 Hannover (DE); Reiter, Rudolf, 31275 Lehrte (DE); Hess, Rüdiger, 30625 Hannover (DE); Neitz, Susanne, 30625 Hannover (DE); Lamping, Norbert, 30625 Hannover (DE); Schorn, Karl, 30625 Hannover (DE)
(74) Representative: Kröncke, Rolf

(57) **Abstract**

Many disorders, especially neoplastic disorders such as cancer and polyps, are the result of hyperproliferative cells or of cells with differentiation disorders. For these types of disorders early detection is of pivotal importance and reliable and early biomarkers for common types of cancers such as colorectal cancer are still not available. Thus, there is a need for new markers and for methods for identifying such new markers. The invention provides peptides and proteins and derivatives thereof originating from transcription intermediary factor-1 beta (TIF1-beta) suitable as markers and therapeutics for disorders such as cancer, preferably colorectal cancerous or related disorders such as polyps.

SEQUENCE LISTING

<110> BioVisioN AG

BioVisioN AG

<120> TIF1-beta peptides and nucleic acids for diagnosis and therapy of cancer
and colorectal cancerous and related disorders

<130> TIF1-b

<160> 11

<170> PatentIn version 3.1

<210> 1

<211> 38

<212> PRT

<213> Homo sapiens

<400> 1

Ala Ala Ser Ala Ala Ala Ala Ser Ala Ala Ala Ala Ser Ala Ala Ser
1               5                   10                  15

Gly Ser Pro Gly Pro Gly Glu Gly Ser Ala Gly Gly Glu Lys Arg Ser
            20                  25                  30

Thr Ala Pro Ser Ala Ala
        35

<210> 2

<211> 39

<212> PRT

<213> Homo sapiens

<400> 2

Met Ala Ala Ser Ala Ala Ala Ala Ser Ala Ala Ala Ala Ser Ala Ala
1               5                   10                  15

Ser Gly Ser Pro Gly Pro Gly Glu Gly Ser Ala Gly Gly Glu Lys Arg

20                  25                  30

Ser Thr Ala Pro Ser Ala Ala
        35

<210>  3

<211>  8

<212>  PRT

<213>  Homo sapiens


<220>

<221>  MISC_FEATURE

<223>  N-terminal extension  r1 and/or C-terminal extension r2 possible;
        r1 and r2 represent sequences which correspond to the sequence o
       r parts of the sequence of the sequence of TIF1-beta from amino a
       cid # 1 to 2 (for r1) and of amino acid # 10 to 39 (for r2)


<400>  3

Ala Ser Ala Ala Ala Ala Ser Ala
1               5

<210>  4

<211>  8

<212>  PRT

<213>  Homo sapiens


<220>

<221>  MISC_FEATURE

<223>  N-terminal extension  r3 and/or C-terminal extension r4 possible;
        r3 and r4 represent sequences which correspond to the sequence o
       r parts of the sequence of the sequence of TIF1-beta from amino a
       cid # 1 to 16 (for r3) and of amino acid # 25 to 39 (for r4)


<400>  4

Ser Gly Ser Pro Gly Pro Gly Glu
1               5

<210>  5

<211>  8

<212>  PRT

<213>  Homo sapiens

<220>

<221>  MISC_FEATURE

<223>  N-terminal extension  r5 and/or C-terminal extension r6 possible;
       r5 and r6 represent sequences which correspond to the sequence o
      r parts of the sequence of the sequence of TIF1-beta from amino a
      cid # 1 to 29 (for r5) and of amino acid # 38 to 39 (for r6)

<400>  5

Glu Lys Arg Ser Thr Ala Pro Ser
1               5

<210>  6

<211>  834

<212>  PRT

<213>  Homo sapiens

<400>  6

Ala Ala Ser Ala Ala Ala Ala Ser Ala Ala Ala Ala Ser Ala Ala Ser
1               5                   10                  15

Gly Ser Pro Gly Pro Gly Glu Gly Ser Ala Gly Gly Glu Lys Arg Ser
            20                  25                  30

Thr Ala Pro Ser Ala Ala Ala Ser Ala Ser Ala Ser Ala Ala Ala Ser
        35                  40                  45

Ser Pro Ala Gly Gly Gly Ala Glu Ala Leu Glu Leu Leu Glu His Cys
    50                  55                  60

Gly Val Cys Arg Glu Arg Leu Arg Pro Glu Arg Glu Pro Arg Leu Leu
65                  70                  75                  80

Pro Cys Leu His Ser Ala Cys Ser Ala Cys Leu Gly Pro Ala Ala Pro
                85                  90                  95

Ala Ala Ala Asn Ser Ser Gly Asp Gly Gly Ala Ala Gly Asp Gly Thr
            100                 105                 110

Val Val Asp Cys Pro Val Cys Lys Gln Gln Cys Phe Ser Lys Asp Ile
        115                 120                 125

Val Glu Asn Tyr Phe Met Arg Asp Ser Gly Ser Lys Ala Ala Thr Asp
    130                 135                 140

Ala Gln Asp Ala Asn Gln Cys Cys Thr Ser Cys Glu Asp Asn Ala Pro
145                 150                 155                 160

Ala Thr Ser Tyr Cys Val Glu Cys Ser Glu Pro Leu Cys Glu Thr Cys
                165                 170                 175

Val Glu Ala His Gln Arg Val Lys Tyr Thr Lys Asp His Thr Val Arg
            180                 185                 190

Ser Thr Gly Pro Ala Lys Ser Arg Asp Gly Glu Arg Thr Val Tyr Cys
        195                 200                 205

Asn Val His Lys His Glu Pro Leu Val Leu Phe Cys Glu Ser Cys Asp
    210                 215                 220

Thr Leu Thr Cys Arg Asp Cys Gln Leu Asn Ala His Lys Asp His Gln
225                 230                 235                 240

Tyr Gln Phe Leu Glu Asp Ala Val Arg Asn Gln Arg Lys Leu Leu Ala
                245                 250                 255

Ser Leu Val Lys Arg Leu Gly Asp Lys His Ala Thr Leu Gln Lys Ser
            260                 265                 270

Thr Lys Glu Val Arg Ser Ser Ile Arg Gln Val Ser Asp Val Gln Lys
        275                 280                 285

Arg Val Gln Val Asp Val Lys Met Ala Ile Leu Gln Ile Met Lys Glu
    290                 295                 300

Leu Asn Lys Arg Gly Arg Val Leu Val Asn Asp Ala Gln Lys Val Thr
305                 310                 315                 320

Glu Gly Gln Gln Glu Arg Leu Glu Arg Gln His Trp Thr Met Thr Lys
                325                 330                 335

Ile Gln Lys His Gln Glu His Ile Leu Arg Phe Ala Ser Trp Ala Leu
            340                 345                 350

Glu Ser Asp Asn Asn Thr Ala Leu Leu Leu Ser Lys Lys Leu Ile Tyr
        355                 360                 365

Phe Gln Leu His Arg Ala Leu Lys Met Ile Val Asp Pro Val Glu Pro
    370                 375                 380

His Gly Glu Met Lys Phe Gln Trp Asp Leu Asn Ala Trp Thr Lys Ser
385                 390                 395                 400

Ala Glu Ala Phe Gly Lys Ile Val Ala Glu Arg Pro Gly Thr Asn Ser
                405                 410                 415

Thr Gly Pro Ala Pro Met Ala Pro Pro Arg Ala Pro Gly Pro Leu Ser
            420                 425                 430

Lys Gln Gly Ser Gly Ser Ser Gln Pro Met Glu Val Gln Glu Gly Tyr
        435                 440                 445

Gly Phe Gly Ser Gly Asp Asp Pro Tyr Ser Ser Ala Glu Pro His Val
    450                 455                 460

Ser Gly Val Lys Arg Ser Arg Ser Gly Glu Gly Glu Val Ser Gly Leu
465                 470                 475                 480

Met Arg Lys Val Pro Arg Val Ser Leu Glu Arg Leu Asp Leu Asp Leu
                485                 490                 495

Thr Ala Asp Ser Gln Pro Pro Val Phe Lys Val Phe Pro Gly Ser Thr
            500                 505                 510

Thr Glu Asp Tyr Asn Leu Ile Val Ile Glu Arg Gly Ala Ala Ala Ala
        515                 520                 525

Ala Thr Gly Gln Pro Gly Thr Ala Pro Ala Gly Thr Pro Gly Ala Pro
    530                 535                 540

Pro Leu Ala Gly Met Ala Ile Val Lys Glu Glu Glu Thr Glu Ala Ala
545                 550                 555                 560

Ile Gly Ala Pro Pro Thr Ala Thr Glu Gly Pro Glu Thr Lys Pro Val
                565                 570                 575

Leu Met Ala Leu Ala Glu Gly Pro Gly Ala Glu Gly Pro Arg Leu Ala
            580                 585                 590

Ser Pro Ser Gly Ser Thr Ser Ser Gly Leu Glu Val Val Ala Pro Glu
        595                 600                 605

Gly Thr Ser Ala Pro Gly Gly Gly Pro Gly Thr Leu Asp Asp Ser Ala
    610                 615                 620

Thr Ile Cys Arg Val Cys Gln Lys Pro Gly Asp Leu Val Met Cys Asn
625                 630                 635                 640

Gln Cys Glu Phe Cys Phe His Leu Asp Cys His Leu Pro Ala Leu Gln
                645                 650                 655

Asp Val Pro Gly Glu Glu Trp Ser Cys Ser Leu Cys His Val Leu Pro
            660                 665                 670

Asp Leu Lys Glu Glu Asp Gly Ser Leu Ser Leu Asp Gly Ala Asp Ser
        675                 680                 685

Thr Gly Val Val Ala Lys Leu Ser Pro Ala Asn Gln Arg Lys Cys Glu
    690                 695                 700

Arg Val Leu Leu Ala Leu Phe Cys His Glu Pro Cys Arg Pro Leu His
705                 710                 715                 720

Gln Leu Ala Thr Asp Ser Thr Phe Ser Leu Asp Gln Pro Gly Gly Thr
                725                 730                 735

Leu Asp Leu Thr Leu Ile Arg Ala Arg Leu Gln Glu Lys Leu Ser Pro
            740                 745                 750

Pro Tyr Ser Ser Pro Gln Glu Phe Ala Gln Asp Val Gly Arg Met Phe
        755                 760                 765

Lys Gln Phe Asn Lys Leu Thr Glu Asp Lys Ala Asp Val Gln Ser Ile
    770                 775                 780

Ile Gly Leu Gln Arg Phe Phe Glu Thr Arg Met Asn Glu Ala Phe Gly
785                 790                 795                 800

Asp Thr Lys Phe Ser Ala Val Leu Val Glu Pro Pro Pro Met Ser Leu
                805                 810                 815

Pro Gly Ala Gly Leu Ser Ser Gln Glu Leu Ser Gly Gly Pro Gly Asp
            820                 825                 830

Gly Pro


<210>  7

<211>  835

<212>  PRT

<213>  Homo sapiens


<400>  7

Met Ala Ala Ser Ala Ala Ala Ala Ser Ala Ala Ala Ala Ser Ala Ala
1               5                   10                  15

Ser Gly Ser Pro Gly Pro Gly Glu Gly Ser Ala Gly Gly Glu Lys Arg
            20                  25                  30

Ser Thr Ala Pro Ser Ala Ala Ala Ser Ala Ser Ala Ser Ala Ala Ala
        35                  40                  45

Ser Ser Pro Ala Gly Gly Gly Ala Glu Ala Leu Glu Leu Leu Glu His
    50                  55                  60

Cys Gly Val Cys Arg Glu Arg Leu Arg Pro Glu Arg Glu Pro Arg Leu
65                  70                  75                  80

Leu Pro Cys Leu His Ser Ala Cys Ser Ala Cys Leu Gly Pro Ala Ala
                85                  90                  95

Pro Ala Ala Ala Asn Ser Ser Gly Asp Gly Gly Ala Ala Gly Asp Gly
            100                 105                 110

Thr Val Val Asp Cys Pro Val Cys Lys Gln Gln Cys Phe Ser Lys Asp
        115                 120                 125

Ile Val Glu Asn Tyr Phe Met Arg Asp Ser Gly Ser Lys Ala Ala Thr
    130                 135                 140

Asp Ala Gln Asp Ala Asn Gln Cys Cys Thr Ser Cys Glu Asp Asn Ala
145                 150                 155                 160

Pro Ala Thr Ser Tyr Cys Val Glu Cys Ser Glu Pro Leu Cys Glu Thr
                165                 170                 175

Cys Val Glu Ala His Gln Arg Val Lys Tyr Thr Lys Asp His Thr Val
            180                 185                 190

Arg Ser Thr Gly Pro Ala Lys Ser Arg Asp Gly Glu Arg Thr Val Tyr
        195                 200                 205

Cys Asn Val His Lys His Glu Pro Leu Val Leu Phe Cys Glu Ser Cys
    210                 215                 220

Asp Thr Leu Thr Cys Arg Asp Cys Gln Leu Asn Ala His Lys Asp His
225                 230                 235                 240

Gln Tyr Gln Phe Leu Glu Asp Ala Val Arg Asn Gln Arg Lys Leu Leu
                245                 250                 255

Ala Ser Leu Val Lys Arg Leu Gly Asp Lys His Ala Thr Leu Gln Lys
            260                 265                 270

Ser Thr Lys Glu Val Arg Ser Ser Ile Arg Gln Val Ser Asp Val Gln
        275                 280                 285

Lys Arg Val Gln Val Asp Val Lys Met Ala Ile Leu Gln Ile Met Lys
    290                 295                 300

Glu Leu Asn Lys Arg Gly Arg Val Leu Val Asn Asp Ala Gln Lys Val
305                 310                 315                 320

Thr Glu Gly Gln Gln Glu Arg Leu Glu Arg Gln His Trp Thr Met Thr
                325                 330                 335

Lys Ile Gln Lys His Gln Glu His Ile Leu Arg Phe Ala Ser Trp Ala
            340                 345                 350

Leu Glu Ser Asp Asn Asn Thr Ala Leu Leu Leu Ser Lys Lys Leu Ile
        355                 360                 365

Tyr Phe Gln Leu His Arg Ala Leu Lys Met Ile Val Asp Pro Val Glu
    370                 375                 380

Pro His Gly Glu Met Lys Phe Gln Trp Asp Leu Asn Ala Trp Thr Lys
385                 390                 395                 400

Ser Ala Glu Ala Phe Gly Lys Ile Val Ala Glu Arg Pro Gly Thr Asn
                405                 410                 415

Ser Thr Gly Pro Ala Pro Met Ala Pro Pro Arg Ala Pro Gly Pro Leu
            420                 425                 430

Ser Lys Gln Gly Ser Gly Ser Ser Gln Pro Met Glu Val Gln Glu Gly
        435                 440                 445

Tyr Gly Phe Gly Ser Gly Asp Asp Pro Tyr Ser Ser Ala Glu Pro His
    450                 455                 460

Val Ser Gly Val Lys Arg Ser Arg Ser Gly Glu Gly Glu Val Ser Gly
465                 470                 475                 480

Leu Met Arg Lys Val Pro Arg Val Ser Leu Glu Arg Leu Asp Leu Asp
                485                 490                 495

Leu Thr Ala Asp Ser Gln Pro Pro Val Phe Lys Val Phe Pro Gly Ser
            500                 505                 510

Thr Thr Glu Asp Tyr Asn Leu Ile Val Ile Glu Arg Gly Ala Ala Ala
        515                 520                 525

Ala Ala Thr Gly Gln Pro Gly Thr Ala Pro Ala Gly Thr Pro Gly Ala
    530                 535                 540

Pro Pro Leu Ala Gly Met Ala Ile Val Lys Glu Glu Glu Thr Glu Ala
545                 550                 555                 560

Ala Ile Gly Ala Pro Pro Thr Ala Thr Glu Gly Pro Glu Thr Lys Pro
                565                 570                 575

Val Leu Met Ala Leu Ala Glu Gly Pro Gly Ala Glu Gly Pro Arg Leu
            580                 585                 590

Ala Ser Pro Ser Gly Ser Thr Ser Ser Gly Leu Glu Val Val Ala Pro
        595                 600                 605

Glu Gly Thr Ser Ala Pro Gly Gly Gly Pro Gly Thr Leu Asp Asp Ser
    610                 615                 620

Ala Thr Ile Cys Arg Val Cys Gln Lys Pro Gly Asp Leu Val Met Cys
625                 630                 635                 640

Asn Gln Cys Glu Phe Cys Phe His Leu Asp Cys His Leu Pro Ala Leu
                645                 650                 655

Gln Asp Val Pro Gly Glu Glu Trp Ser Cys Ser Leu Cys His Val Leu
            660                 665                 670

Pro Asp Leu Lys Glu Glu Asp Gly Ser Leu Ser Leu Asp Gly Ala Asp
        675                 680                 685

Ser Thr Gly Val Val Ala Lys Leu Ser Pro Ala Asn Gln Arg Lys Cys
    690                 695                 700

Glu Arg Val Leu Leu Ala Leu Phe Cys His Glu Pro Cys Arg Pro Leu
705                 710                 715                 720

His Gln Leu Ala Thr Asp Ser Thr Phe Ser Leu Asp Gln Pro Gly Gly
                725                 730                 735

Thr Leu Asp Leu Thr Leu Ile Arg Ala Arg Leu Gln Glu Lys Leu Ser
            740                 745                 750

Pro Pro Tyr Ser Ser Pro Gln Glu Phe Ala Gln Asp Val Gly Arg Met
        755                 760                 765

Phe Lys Gln Phe Asn Lys Leu Thr Glu Asp Lys Ala Asp Val Gln Ser
    770                 775                 780

Ile Ile Gly Leu Gln Arg Phe Phe Glu Thr Arg Met Asn Glu Ala Phe
785                 790                 795                 800

Gly Asp Thr Lys Phe Ser Ala Val Leu Val Glu Pro Pro Pro Met Ser
                805                 810                 815

Leu Pro Gly Ala Gly Leu Ser Ser Gln Glu Leu Ser Gly Gly Pro Gly
            820                 825                 830

Asp Gly Pro
        835

<210>  8

<211>  2989

<212>  DNA

<213>  Homo sapiens

<400> 8
ggcgcgcggg cgagcggttg tgcttgtgct tgtggcgcgt ggtgcgggtt tcggcggcgg 60
ctgaggaaga agcgcgggcg gcgccttcgg gaggcgagca ggcagcagtt ggccgtgccg 120
tagcagcgtc ccgcgcgcgg cgggcagcgg cccaggaggc gcgtggcggc gctcggcctc 180
gcggcggcgg cggcggcagc ggcccagcag ttggcggcga gcgcgtctgc gcctgcgcgg 240
cgggccccgc gcccctcctc ccccctgggc cgcccccggc ggcgtgtgaa tggcggcctc 300
cgcggcggca gcctcggcag cagcggcctc ggccgcctct ggcagcccgg gcccgggcga 360
gggctccgct ggcggcgaaa agcgctccac cgccccttcg gccgcagcct cggcctctgc 420
ctcagccgcg gcgtcgtcgc ccgcgggggg cggcgccgag gcgctggagc tgctggagca 480
ctgcggcgtg tgcagagagc gcctgcgacc cgagagggag ccccgcctgc tgccctgttt 540
gcactcggcc tgtagtgcct gcttagggcc cgcggccccc gccgccgcca acagctcggg 600
ggacggcggg gcggcgggcg acggcaccgt ggtggactgt cccgtgtgca agcaacagtg 660
cttctccaaa gacatcgtgg agaattattt catgcgtgat agtggcagca aggctgccac 720
cgacgcccag gatgcgaacc agtgctgcac tagctgtgag gataatgccc cagccaccag 780
ctactgtgtg gagtgctcgg agcctctgtg tgagacctgt gtagaggcgc accagcgggt 840
gaagtacacc aaggaccata ctgtgcgctc tactgggcca gccaagtctc gggatggtga 900
acgtactgtc tattgcaacg tacacaagca tgaacccctt gtgctgtttt gtgagagctg 960
tgatactctc acctgccgag actgccagct caatgcccac aaggaccacc agtaccagtt 1020
cttagaggat gcagtgagga accagcgcaa gctcctggcc tcactggtga agcgccttgg 1080
ggacaaacat gcaacattgc agaagagcac caaggaggtt cgcagctcaa tccgccaggt 1140
gtctgacgta cagaagcgtg tgcaagtgga tgtcaagatg gccatcctgc agatcatgaa 1200
ggagctgaat aagcggggcc gtgtgctggt caatgatgcc cagaaggtga ctgaggggca 1260
gcaggagcgc ctggagcggc agcactggac catgaccaag atccagaagc accaggagca 1320
cattctgcgc tttgcctctt gggctctgga gagtgacaac aacacagccc ttttgctttc 1380
taagaagttg atctacttcc agctgcaccg ggccctcaag atgattgtgg atcccgtgga 1440
gccacatggc gagatgaagt ttcagtggga cctcaatgcc tggaccaaga gtgccgaggc 1500
ctttggcaag attgtggcag agcgtcctgg cactaactca acaggccctg cacccatggc 1560
ccctccaaga gccccagggc ccctgagcaa gcagggctct ggcagcagcc agcccatgga 1620
ggtgcaggaa ggctatggct ttgggtcagg agatgatccc tactcaagtg cagagcccca 1680
tgtgtcaggt gtgaaacggt cccgctcagg tgagggcgag gtgagcggcc ttatgcgcaa 1740
ggtgccacga gtgagccttg aacgcctgga cctggacctc acagctgaca gccagccacc 1800
cgtcttcaag gtcttcccag gcagtaccac tgaggactac aaccttattg ttattgaacg 1860
tggcgctgcc gctgcagcta ccggccagcc agggactgcg cctgcaggaa cccctggtgc 1920
cccacccctg gctggcatgg ccattgtcaa ggaggaggag acggaggctg ccattggagc 1980

ccctcctact gccactgagg gccctgagac caaacctgtg cttatggctc ttgcggaggg 2040
tcctggtgct gagggtcccc gcctggcctc acctagtggc agcaccagct cagggctgga 2100
ggtggtggct cctgagggta cctcagcccc aggtggtggc ccgggaaccc tggatgacag 2160
tgccaccatt tgccgtgtct gccagaagcc aggcgatctg gttatgtgca accagtgtga 2220
gttttgtttc cacctggact gtcacctgcc ggccctgcag gatgtaccag gggaggagtg 2280
gagctgctca ctctgccatg tgctccctga cctgaaggag gaggatggca gcctcagcct 2340
ggatggtgca gacagcactg gcgtggtggc caagctctca ccagccaacc agcggaaatg 2400
tgagcgtgta ctgctggccc tattctgtca cgaaccctgc cgccccctgc atcagctggc 2460
taccgactcc accttctccc tggaccagcc cggtggcacc ctggatctga ccctgatccg 2520
tgcccgcctc caggagaagt tgtcacctcc ctacagctcc ccacaggagt ttgcccagga 2580
tgtgggccgc atgttcaagc aattcaacaa gttaactgag gacaaggcag acgtgcagtc 2640
catcatcggc ctgcagcgct tcttcgagac gcgcatgaac gaggccttcg gtgacaccaa 2700
gttctctgct gtgctggtgg agcccccgcc gatgagcctg cctggtgctg gcctgagttc 2760
ccaggagctg tctggtggcc ctggtgatgg cccctgaggc tggagccccc atggccagcc 2820
cagcctggct ctgttctctg tcctgtcacc ccatccccac tcccctggtg gcctgactcc 2880
cactccctgg tggccccatc ccccagttcc tcacgatatg gtttttactt ctgtggattt 2940
aataaaaact tcaccagtta aaaaaaaaaa aaaaaaaaaa aaaaaaaaa 2989

<210> 9

<211> 35

<212> PRT

<213> Homo sapiens


<400> 9

Ser Asp Ala Ala Val Asp Thr Ser Ser Glu Ile Thr Thr Lys Asp Leu
1               5                   10                  15


Lys Glu Lys Lys Glu Val Val Glu Glu Ala Glu Asn Gly Arg Asp Ala
            20                  25                  30


Pro Ala Asn
        35

<210> 10

<211> 36

<212> PRT

<213> Homo sapiens

<400>  10

Ser Asp Ala Ala Val Asp Thr Ser Ser Glu Ile Thr Thr Lys Asp Leu
1               5                   10                  15

Lys Glu Lys Lys Glu Val Val Glu Glu Ala Glu Asn Gly Arg Asp Ala
            20                  25                  30

Pro Ala Asn Gly
        35

<210>  11

<211>  21

<212>  PRT

<213>  Homo sapiens

<400>  11

Thr Arg Arg Ile Val Asp Gly Lys Val Val Ser Glu Thr Asn Asp Thr
1               5                   10                  15

Lys Val Leu Arg His
            20

## Description

### FIELD OF THE INVENTION

The present invention is in the field of diagnosis and therapy of diseases or disorders due to cells malfunctioning in proliferation and/or differentiation. In particular, the present invention relates to the diagnosis of cancer, preferably colorectal cancerous and related disorders. Especially the invention is directed to peptides and nucleic acids corresponding to TIF1-beta and other molecules which are released from cells with a hyperproliferative disorder, or from cells with a differentiation disorder, or from cells with a neoplastic disorder. Many kinds of these cells represent tumor or polyp cells. The peptides and nucleic acids of the invention were identified by transplanting a xenograft into immune-compromised animals and searching for peptides released from the implanted cells, among others by analyzing the plasma of said animals. These peptides and nucleic acids are potential markers for the implanted cells, which represent models for cells such as tumor cells or cells of polyps. Preferably, the markers according to the present invention are markers for tumor or polyp cells present in the colon or rectum. Furthermore the invention is directed to methods to isolate, methods to detect in biological or diagnostic samples, test kits for the detection of markers or biomarkers and methods to use these peptides and their corresponding nucleic acids in therapy.

### BACKGROUND OF THE INVENTION

Many diseases and disorders, especially cancer, are due to cells with hyperproliferative disorders or cells with differentiation disorders. For these kinds of disorders early detection is of pivotal importance. However, reliable and early biomarkers for the most common types of cancers such as breast, prostate, lung and colorectal cancer are still not available. Thus, there is a need for new markers and for methods for identifying such new markers.

Transcriptional repression plays an important role in many processes of development and differentiation as well as in cancer and related disorders. Regulatory genes encoding sequence-specific transcriptional repressors often contain a DNA-binding domain and one or more repression domains. These repression domains exert their function by interactions with transcriptional intermediary factors (TIFs). These TIFs function by remodeling the chromatin structure, by inhibiting the (pre)initiation complex, or by associating target genes with specialized nuclear compartments that confers transcriptional repression (Development, 2000, 127:2955-2963).

One of these TIFs is TIF1-beta. For TIF1-beta there are used various other names in the prior art such as Transcription Intermediary Factor 1-beta, TIF1β, TF1B, Tif1 B, Tripartite Motif Protein 28, TRIM28, Tripartite Motif-Containing 28, Nuclear Corepressor KAP-1, KAP-1, KRIP-1, or KRAB-associated protein 1. The KRAB (Krüppel-associated box) domain, a protein domain of about 75 amino acids length, is a common transcriptional repression domain of zinc-finger proteins. TIF1-beta interacts with the KRAB domain and enhances KRAB-mediated repression. TIF1-beta is also known to be associated with members of the heterochromatin protein 1 family, which protein family is known to function in Drosophila in gene silencing (Development, 2000, 127:2955-2963). TIF1-beta belongs to the TIF1 protein family, which in addition comprises TIF1-alpha, a putative nuclear receptor cofactor, and TIF1-gamma, a protein of unknown functions. Neither TIF1-alpha, nor TIF1-gamma interact with or act as a corepressor for several members of the KRAB-zinc finger protein family. TIF1-beta is essential for early embryonic development resulting in the death of TIF1-beta deficient mouse embryos (Development, 2000, 127:2955-2963). TIF1-beta or TIF1-beta fragments never have been shown or used as a marker or therapeutic agent of cancer.

### SUMMARY OF THE INVENTION

A first aspect of the invention provides a method for the detection of the presence or absence of cells in an individual, wherein said cells are chosen from the group consisting of cells having a hyperproliferative disorder, cells having a differentiation disorder and neoplastic cells comprising the steps of determining the amount of TIF1-beta and/or of a TIF1-beta fragment and/or derivatives and/or polymorphic forms thereof in a sample of said individual, comparing the result of the first step with the result determined using a negative control sample or with a known reference value and determining the presence or absence of said cells in said individual based on the result of the comparison made. Preferably the TIF1-beta fragments selected for this comparison are selected from the group consisting of TIF1-beta fragments according to Seq.-ID 1 to 5 and/or derivatives and/or polymorphic forms thereof, the cells detected using this method are preferably cancer cells, more preferably the cells are cells having a colorectal cancerous or a related disorder, preferably the sample used for this method is selected from the group consisting of whole blood, plasma, serum, cerebrospinal fluid, lymph, urine, stool and an tissue sample, preferably the measurement method used for determining said TIF1-beta and/or said fragment of TIF1-beta and/or a derivative and/or polymorphic form thereof is selected form the group consisting of ELISA, RIA, western blot, protein chip assays, mass spectrometry, immune histology, flow cytometry or by molecular biologic methods

A second aspect of the invention are peptides, which peptides comprise a fragment of TIF1-beta and/or a derivatives and/or a polymorphic forms thereof, wherein said peptides are absent or present in altered quantities in an individual, and wherein said individual comprises cells chosen from the group consisting of cells having a hyperproliferative disorder, cells having a differentiation disorder and neoplastic cells and the result is compared to the quantities of said peptides in an individual not comprising said cells, with the proviso that said peptides are not chosen from the group consisting of Seq.-ID 6 or 7. Preferably said individual comprises cancer cells, more preferably cells having a colorectal cancerous or a related disorder. Preferably said fragments of TIF1-beta claimed have a sequence according to any one of Seq.-IDs 1 to 5. Preferably the peptides claimed may comprise at least one additional non-TIF1-beta amino acid sequence.

A further aspect of the invention is a method to identify TIF1-beta fragments and/or derivatives and/or polymorphic forms thereof, which TIF1-beta fragments are present in a sample of the organism of a host comprising a xenograft, wherein said xenograft releases said TIF1-beta fragments or wherein said xenograft releases precursors of said TIF1-beta fragment which are subsequently, comprising the steps of implanting a xenograft into the organism of a non-human host and letting grow said xenograft for a certain amount of time within said organism of said non-human host, collecting a sample from said organism of a non-human host, determining the presence, absence or quantity of a plurality of peptides present in said sample and identifying those peptides, which are absent or which are present in altered quantities in a sample of a non-human host not transplanted with said xenograft and which peptides are TIF1-beta fragments and/or a derivatives and/or a polymorphic forms thereof.

Furthermore the invention includes TIF1-beta fragments and/or derivatives and/or polymorphic forms thereof identified with the method described in the previous sentence, with the proviso that the peptides according to Seq.-ID 6 and 7 are not included, and preferably said fragments of TIF1-beta claimed have a sequence according to any one of Seq.-IDs 1 to 5 and preferably the peptides claimed may comprise at least one additional non-TIF1-beta amino acid sequence.

A further aspect of the invention are binding agents specifically binding to a neoEpitope of a peptide according to Seq.-ID 1 to 5, which binding agents do not bind a peptide according to Seq.-ID 6 or 7 (TIF1-beta with or without N-terminal methionine). Preferably these binding agents are antibodies, fragments or derivatives thereof.

A still further aspect of the invention relates to nucleic acids selected from the group consisting of
a) a nucleic acid sequence encoding a peptide of the invention;
b) a nucleic acid sequence which is complementary to a nucleic acid sequence encoding a peptide of the invention;
c) a nucleic acid sequence, which hybridizes under stringent conditions to a nucleic acid under a) and/or b) and which at maximum has a nucleic acid sequence length which is shorter than the nucleic acid sequence coding for Seq.-ID 6 or 7;
d) a nucleic acid sequence, which is degenerated with respect to said nucleic acid sequence under any one of a) to c);
e) a derivative and/or fragment of a nucleic acid sequence under any one of a) to d);
f) a nucleic acid sequence according to a), b), c), d) or e) further comprising at least one non-TIF1-beta nucleic acid sequence;
g) a nucleic acid sequence according to f), wherein said at least one non-TIF1-beta nucleic acid sequence is a sequence for labeling of the nucleic acid sequence.

Preferably the label of the nucleic acid sequence is not a nucleic acid sequence.

A further aspect of the invention is a vector comprising a nucleic acid of the invention and a host cell comprising a nucleic acid of the invention or comprising a vector of the invention.

Another embodiment of the invention is a test kit or a pharmaceutical composition comprising a peptide according to the invention, and/or a binding agent according to the invention, and/or a nucleic acid according to the invention, and/or a vector according to the invention, and/or a host cell according to the invention and instructions how to use the test kit for determining the presence, absence, prognosis or relapse of a disorder.

Another embodiment of the invention is a test kit with instructions how to use said test kit for a method according to the invention, or a pharmaceutical composition for treatment and/or prophylaxis and/or diagnosis of a disease selected from the group consisting of cancer, colorectal cancerous or related disorders, wherein the test kit or the pharmaceutical composition is comprising
a) a peptide according to the invention; and/or
b) a peptide which is TIF1-beta or a fragment and/or a derivative and/or a polymorphic form thereof; and/or
c) a peptide according to b) which in addition comprises at least one non-TIF1-beta amino acid; and/or
d) a binding agent according to the invention; and/or
e) a binding agent specific for TIF1-beta or specific for a fragment or derivative or polymorphic form thereof; and/or
f) a nucleic acid according to the invention; and/or
g) a nucleic acid which is coding for TIF1-beta and/or a fragment and/or derivative and/or a polymorphic form thereof; and/or
h) a nucleic acid sequence which is complementary to or which hybridizes under stringent conditions to a nucleic acid according to the invention; and/or
i) a nucleic acid according to the invention which in addition comprises at least one non-TIF1-beta nucleotide; and/or
j) a vector according to the invention; and/or
k) a host cell according to the invention.

Another aspect of the invention is the use of the materials according to any one of a) to k), as described above, for the manufacture of a test kit and/or for the manufacture of a pharmaceutical composition for treatment and/or prophylaxis and/or for diagnosis of a disease selected from the group consisting of cancer, colorectal cancerous or related disorders.

Another embodiment of the invention is the use of a peptide according to the invention, and/or a binding agent according to the invention, and/or a nucleic acid according to the invention, and/or a vector according to the invention, and/or a host cell according to the invention and instructions how to use the test kit for determining the presence, absence, prognosis or relapse of a disorder for the manufacture of a test kit or a pharmaceutical composition.

### BRIEF DESCRIPTION OF THE DRAWINGS

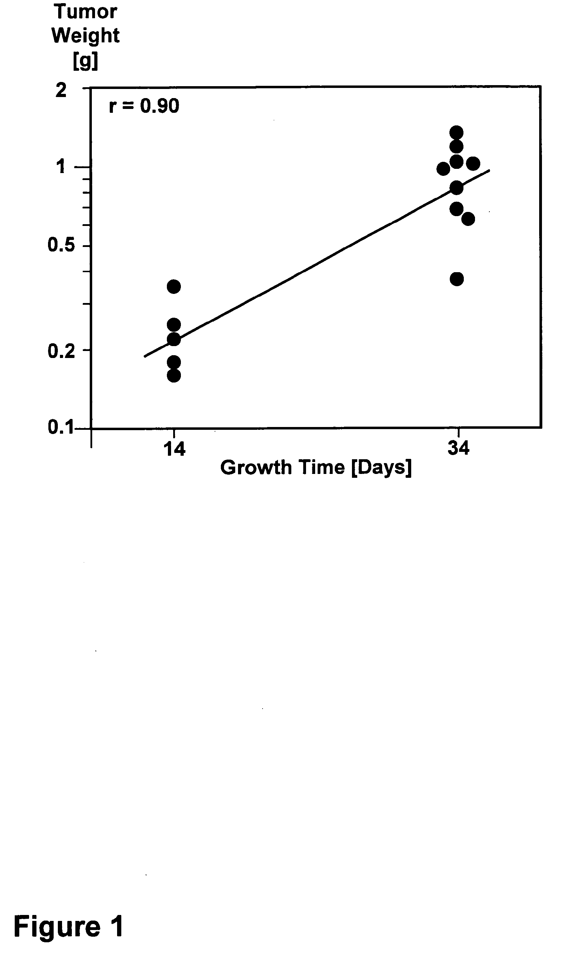

Figure 1: Figure 1 shows the tumor weight (y-axis) as a function of growth time of HCT-116 tumor cells (x-axis). The tumor weight is depicted in gram; the growth time of the tumor is given in days. The measurement values of individual tumors, measured on day 14 or on day 34 post tumor cell injection, are depicted as bullets. The correlation r of time versus tumor weight was 0.90.

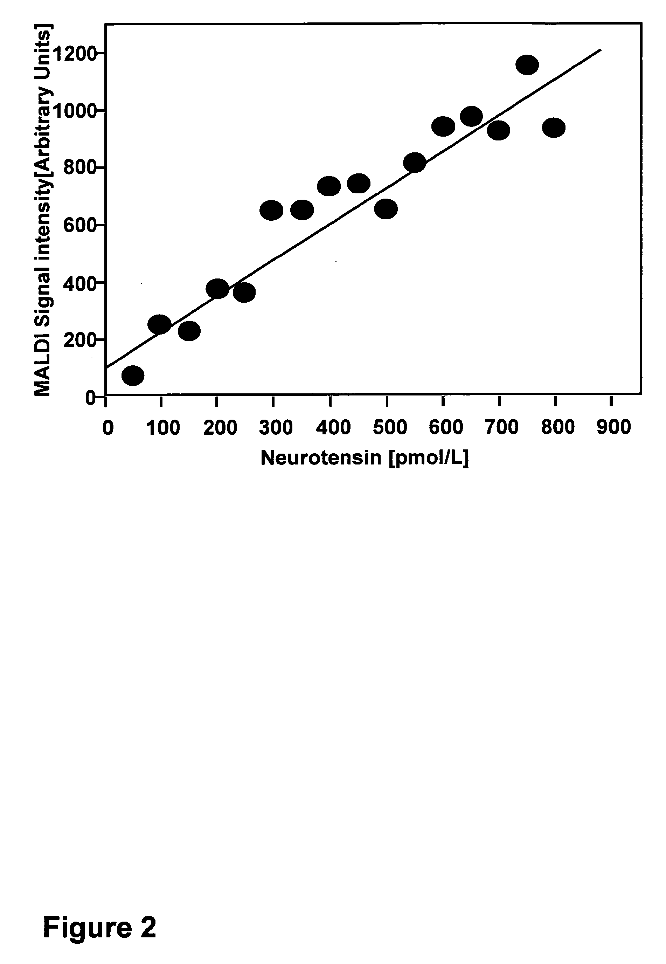

Figure 2: 50 to 800 pmol neurotensin were introduced into plasma samples, subsequently separated by reversed phase chromatography and those fractions containing the neurotensin were analyzed by mass spectrometry as described in the examples. There is a the linear correlation between the MALDI mass spectrometry mass signal intensity (y-axis) of the neurotensin peptide and the amount of the neurotensin peptide added to the plasma sample (x-axis), indicating that MALDI mass spectrometry is suitable to quantitatively determine peptides in complex samples such as plasma.

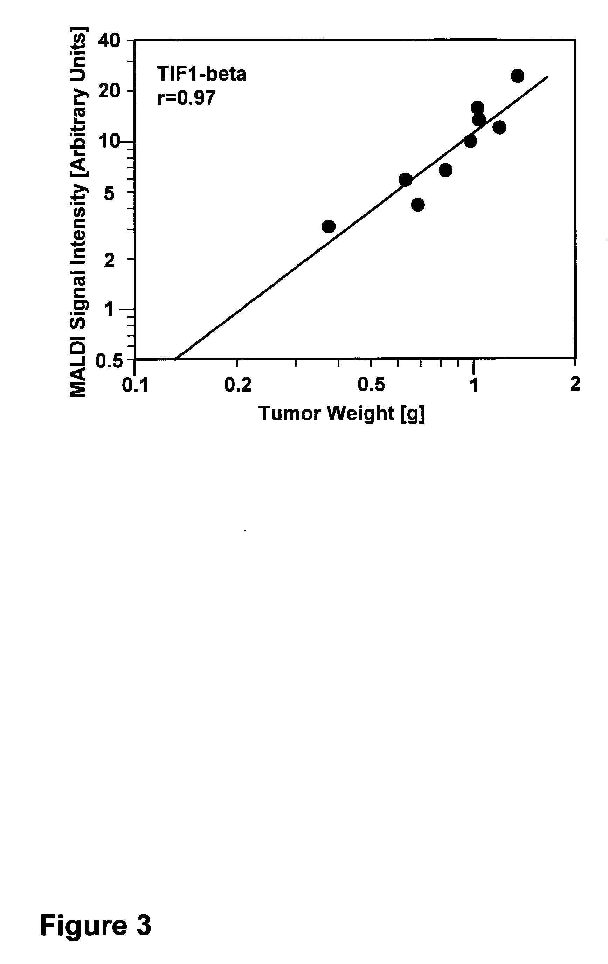

**Figure 3:** This figure shows the MALDI mass signal intensity of the peak of the TIF1-beta fragment (found in plasma) corresponding to amino acid residues 2 to 39 of TIF1-beta (Table 1, Seq.-ID 1), versus the wet weight of the tumors in individual mice. The x- and y-axis are in logarithmic format. The y-axis represents the MALDI mass signal intensity in arbitrary units, whereas the x-axis represents the tumor weight. The correlation coefficient r of the MALDI mass signal intensity versus the tumor mass was 0.97.

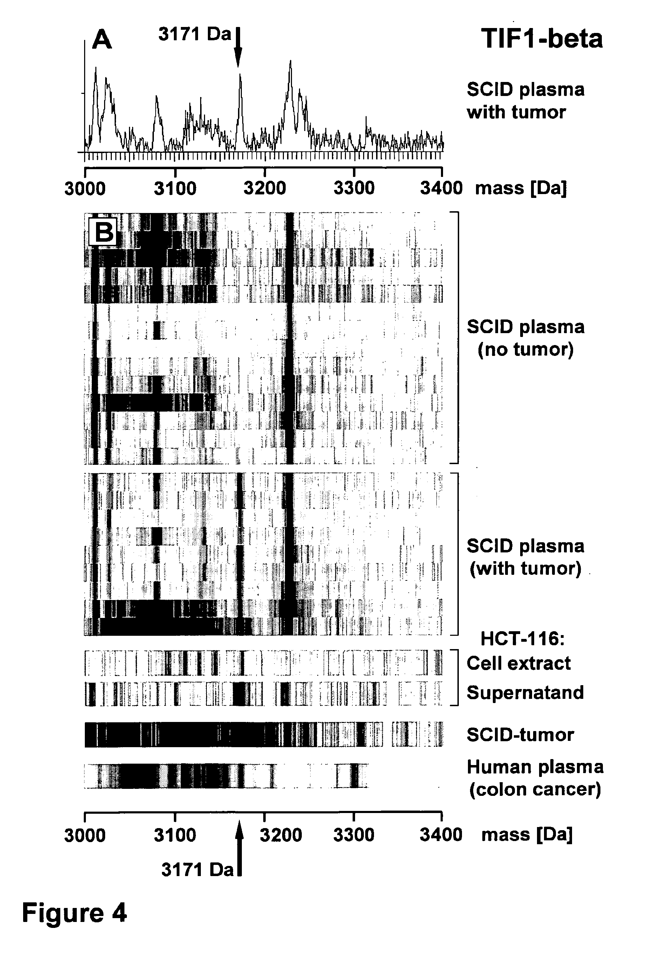

Figure 4: Panel A shows a mass spectrum of an individual fraction obtained by RP-chromatography of SCID-mice plasma, 34 days after injection of the HCT-116 colon tumor cells. The y-axis represents the MALDI mass signal intensity and the x-axis represents the molecular mass in Dalton. An arrow indicates the peak representing a peptide of interest (experimentally determined mass was 3171 Dalton; the theoretical monoisotopic mass is 3169 Dalton), which, after sequencing, was identified as a fragment of human TIF1-beta (TIF1-b) corresponding to amino acid residues 2 to 39 of TIF1-beta. Panel B shows the same mass window as panel A of various mass spectra generated using different samples. These mass spectra are depicted in a "gel-like" view, wherein the mass signal intensities are converted into color intensity (gray scale), to enable an easier comparison of the samples. The MALDI mass signal intensities of the following samples are shown in the following order: 14 individually measured plasma samples of 14 individual SCID mice without a HCT-116 tumor, 9 individually measured plasma samples of 9 individual SCID mice having a HCT-116 tumor, 1 sample of a cell extract prepared from in vitro cultured HCT-116 cells, 1 sample of the supernatant collected from in vitro cultured HCT-116 cells, 1 sample of a pool of human plasma collected from 19 colon carcinoma patients. The arrow at the bottom shows the same TIF1-beta fragment, which is shown in panel A. The MALDI mass signal of this TIF-beta peptide is present in all samples, except in the plasma samples of SCID mice without a HCT-116 tumor.

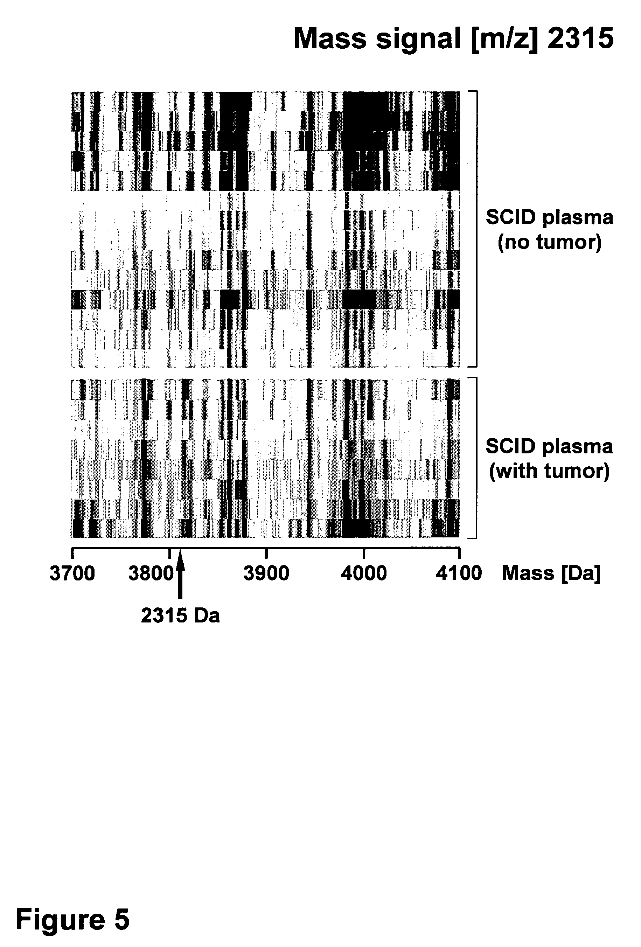

Figure 5: Figure 5 shows the same kind of experiment as figure 4. All samples are depicted in a "gel-like" view. The MALDI mass signal intensities of the following samples are shown in the following order: 14 individually measured plasma samples of SCID mice without a HCT-116 tumor, 8 individually measured plasma samples of SCID mice having a HCT-116 tumor. The arrow at the bottom of the figure indicates an experimental determined mass signal of 2315 Dalton (identified in fraction 35), corresponding to a peptide of unknown sequence. This mass signal is present in plasma samples of SCID mice with a HCT-116 tumor, but is absent or very low in plasma samples of SCID mice without a HCT-116 tumor.

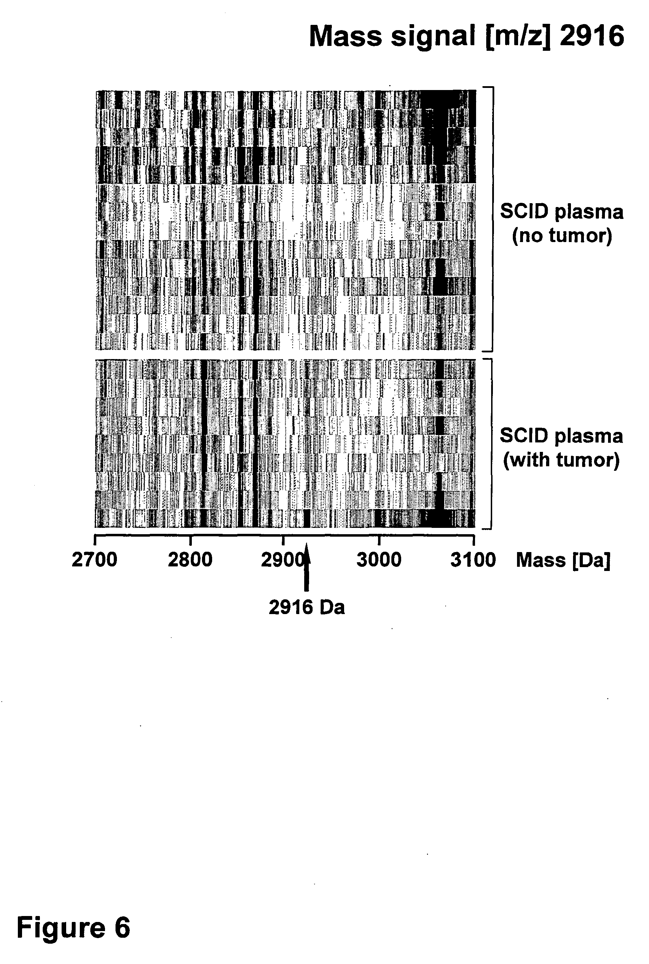

Figure 6: This figure shows the same kind of experiment as figure 4 and 5. All samples are depicted in a "gel-like" view. The MALDI mass signal intensities of the following samples are shown in the following order: 14 individually measured plasma samples of SCID mice without a HCT-116 tumor, 9 individually measured plasma samples of SCID mice having a HCT-116 tumor. The arrow at the bottom of the figure indicates an experimental determined mass signal of 2916 Dalton (identified in fraction 35), corresponding to a peptide of unknown sequence. This mass signal is present in plasma samples of SCID mice with a HCT-116 tumor, but is absent or very low in plasma samples of SCID mice without a HCT-116 tumor.

### DETAILED DESCRIPTION OF THE INVENTION

In one embodiment, the present invention is directed to the use of experimental animals, preferably immune-compromised non-human hosts, preferably immune-compromised non-human mammals, preferably in immune-compromised rodents such as rats or mice, preferably SCID mice, which have implanted a xenograft comprising cells with a hyperproliferative disorder or with a differentiation disorder or neoplastic cells, preferably a xenograft of human tissue or cells, preferably human neoplastic tissue or cells, preferably human colorectal neoplastic tissue or cells or the colon carcinoma cell line HCT-116 for identifying molecules such as peptides and/or proteins suitable as marker for the presence of the above mentioned cells. Using proteomics and peptidomics® methods such as mass spectrometry the composition of peptides and/or proteins of for example a plasma sample of the experimental animal were analyzed. To be suitable for example as a marker molecule, the identified peptides and/or proteins originate from the xenograft and not from the experimental animal. It is also possible to compare the peptide and/or protein composition of for example a plasma sample from the organism of an experimental animal transplanted with tumor cells with the peptide and/or protein composition of another experimental animal transplanted with control cells, which control cells are different to the tumor cells.

One advantage of such a marker search strategy is that peptides and/or proteins originating from the xenograft can easily be distinguished from those originating from the experimental animal, as the sequences are from a species different to the species of the experimental animal.

Another advantage of the invention is the use of immune-compromised experimental animals (hosts), which allows the xenograft to grow in the organism of the experimental animal without being subjected to the attacks of the immune system of the host. This allows the analysis of almost any kind of cells such as for example neoplastic cells. In addition the immune-compromised experimental animals do not develop inflammatory reactions as a consequence of the xenograft. Consequently the peptides and/or proteins of the invention therefore are not the result of such an inflammatory reaction. This is a big advantage, as inflammatory reactions take place in many different disorders, not just in for example neoplastic disorders and thus markers reflecting inflammation are usually not very specific markers.

The peptides and/or proteins identified in the invention as potential markers of, for example, a neoplastic disorder, were subjected to various "environmental factors" within the organism of the experimental animal, which is a further advantage of the peptides of the invention. This ensures that predominantly those peptides (markers, biomarkers) are identified, which survive the typical "environmental factors", or which are generated as a consequence of these "environmental factors". Markers which are very susceptible to proteolytic degradation, or which stick very well to abundant plasma proteins such as albumin, or which are efficiently removed from the circulation being bound to proteins and/or receptors or which are excreted efficiently by the kidneys are not likely to be present in the experimental animal in high concentrations. Therefore these markers are less likely to be identified in this invention and exactly these markers are also less suitable as marker for disorders such as neoplastic disorders. So the use of experimental animals selectively enriched those peptides and/or proteins which in vivo survive well and therefore are more suitable as diagnostic markers. The organism of the experimental animal simulates the fate of potential markers in the organism of a patient. Therefore the identified markers, such as TIF1-beta fragments represent markers which most likely are suitable for use in for example in human cancer patients.

Another advantage of the invention is that many additional characteristics of for example disorders resulting from cells with a hyperproliferative disorder and/or resulting from cells with a differentiation disorder and/or resulting from neoplastic cells, i.e. cells malfunctioning in proliferation and differentiation, can be analyzed and as a consequence markers with special, advantageous characteristics can be identified. For example it is possible to identify markers, which correlate with certain properties or characteristics of a tumor (neoplastic cells) such as the size of the tumor, the physical location of the tumor, the tendency of the tumor to form metastases, the type of the tumor for example a colon tumor which is positive for certain receptors, etc. All of these types of markers generally cannot be identified using other methods which use in vitro grown tumor cells for marker identification. On the other hand getting tumor samples from human patients with defined certain properties or characteristics is difficult, as it is ethical not justifiable to manipulate or treat human patients like experimental animals. In addition human patients are much more diverse regarding their phenotype, than genetically nearly identical inbred experimental animals housed under standardized conditions. Therefore experimental studies directly working with human patient samples comprise much more uncertain and unknown variables which may hide markers, which are detectable, if the method described in this invention is used.

A further advantage of the markers of the invention is the possibility that the identified markers of cells with a hyperproliferative disorder and/or cells with a differentiation disorder and/or neoplastic cells, may also be suitable to predict the success of a treatment to remove, kill or suppress the growth or viability of said cells within a living organism such as a patient. With viability is meant that cells are still alive, but do not proliferate at all or do proliferate at a lower than usual rate. Therefore it is possible to identify with the methods described in this invention markers for monitoring the therapeutic success or even for predicting a therapeutic success even before the treatment has started. This can be done in general with certain types of cells with a hyperproliferative disorder and/or certain types of cells with a differentiation disorder and/or certain types of neoplastic cells, but it also can be done with purified cells or tissues of a certain, individual patient, thereby identifying a tailor-made, patient-specific marker for a disorder or for predicting the therapy success or for monitoring the potential relapse of the treated disorder in an individual patient.

### Experimental animals / immune-compromised host

Preferably the experimental animals used are non-human hosts, non-human mammals, preferably rodents, preferably mice or rats, preferably animals with impaired immune system e.g. immune-compromised animals, regardless of the reason for the impairment of the immune system. With immune-compromised animals is meant any kind of animal with an immune system which is suppressed to such an extend, that xenografts from a species different to said animal, preferably xenografts originating from human cells, human tissues, human organs, human tumors, etc., survive for longer periods of time within the organism of said animal without being destroyed by the immune system of said immune-compromised animal. With longer periods of time is meant at least 1 day, preferably 2, 3, 4, 5, 6, 7, 14, 21, 28 days, 1, 2, 3, 4, 5 or 6 months or even longer periods of time. Depending on the growth-rate and on the number of cells or the amount of tissue or the size of the tumor or organ representing the xenograft, different periods of times are possible. Fast growing xenografts can be grown only for shorter periods of time ranging from days to several weeks, whereas slowly growing or not growing xenografts can be present within the organism of the animal for longer periods of time in the range of several months. The animal can be of any species, but species commonly used in laboratories such as mice, rats, guinea pigs, rabbits, goats, pigs, monkeys, dogs, cats, etc. are preferred.

The reason for the impaired immune system among others can be a natural genetic defect of the animal, a disorder of the animal or an experimental treatment of the animal. Experimental treatments among others can be surgical modifications of the animal, physical treatment of the animal, genetic manipulation of the animal or the treatment of the animal with substances inhibiting or destroying the immune system of the animal. An example of a surgical modification resulting in an immune-compromised experimental animal is the surgical removal of the thymus. An example of a physical treatment of animals, resulting in an compromised immune system is the destruction of the bone marrow by irradiation or by sub lethal irradiation of the whole animal. Genetic defects of the experimental animal can be caused by mutations present in nature or by experimentally induced random or specific mutations of the animal resulting in for example a genetic knockout animal or a transgenic animal. Examples of genetic defects, resulting in a compromised immune system are SCID mice (severe combined immunodeficiency), Prkdc (protein kinase DNA activated catalytic polypeptide gene defect), nude mice and rats (Foxn1 (forkhead box N1) defect), Rag1 mice (Rag1 = recombination activating gene 1), beige mice (bg<J> mutation and animals of other species with a deletion of at least one of the genes selected from the group comprising of Foxn1, Prkdc, Rag1, bg<J> gene, MHC II (major histocompatibility cluster II) and ADA (adenosine desaminase). These experimental animals with genetic defects are for example available from the Jackson Laboratories, Bar Harbor, ME, USA or from Taconic Farms, Germantown NY, USA. It is possible to combine two or more immune-compromised animal models, for example by cross-breeding of different genetically immune-compromised mice strains, or by irradiation of a normal rat resulting in complete destruction of its bone marrow and subsequent transplantation of for example bone marrow of SCID mice to the irradiated rat resulting in some kind of a SCID-like rat (Transplantation, 1997, 64:1541-50). Furthermore certain viruses result in immune-compromised animals such as for example the simian immunodeficiency virus or the simian/human immunodeficiency virus and consequently experimental animals infected with such viruses can be used according to the invention. Substances inhibiting the immune system (immunosuppressants) can be used to enable the survival and/or growth of xenografts according to the invention. Examples of such drugs are among others cyclosporine, cortisone, prednisolone, azathioprine, cyclophosphamide, tacrolimus, prednisone, mycophenolate and sirolimus. Also certain antibodies such as anti-CD3 antibodies, which inhibit T-cell function (muromonab-CD3) can be used as immunosuppressants to enable survival and/or growth of xenografts in experimental animals.

### Alternatives to immune-compromised animals

Instead of using immune-compromised experimental animals it is possible to grow the xenograft in a compartment within the experimental animal, which is physically separated from the cells, especially the immune cells, of the experimental animal. This can be achieved by implanting for example cells fixed in a gel or other matrix such as matrigel® from Discovery Labware, Bedford, MA, USA, which ensures that the cells have no physical contact to the immune cells of the hosts organism but at the same time are supplied with metabolites by the host and in reverse can release substances which can diffuse through the gel or matrix into the organism of the host. Alternatively to using a gel or matrix compartments which are connected to the organism of the host, for example by use of a membrane can be used, wherein the pore size of the membrane is small enough to prevent passage of cells, but big enough to allow diffusion of peptides, proteins and metabolites. In general all kinds of compartments and methods can be used, which ensure, that peptides and/or proteins and metabolites can reach and leave the xenograft as long as at the same time there is now physical contact between the cells of the xenograft and the cells of the host. These compartments can be present within the organism of the experimental animal or they can be present outside of the organism of the animal. If these compartments are outside of the organism of the experimental animal they for example can be connected to the organism of the experimental animal by use of a tubing connecting the blood circulation of the animal to the compartment but still preventing the cells of the blood from entering a chamber within the compartment, which comprises the xenograft.

### Hyperproliferative disorders, differentiation disorders and neoplastic disorders

The method used to identify the peptides of the invention in general can be used to identify markers of cells with a hyperproliferative disorder and/or a differentiation disorder and/or a neoplastic disorder. With hyperproliferative disorder is meant that the proliferation of the cells is not tightly regulated like in normal, healthy cells resulting in uncontrolled growth of the hyperproliferative cells. This results often in destruction and suppression of healthy nearby cells and tissues. Normal, healthy cells are usually limited in their proliferation rate and their growth within a tissue by contact to other cells which is termed contact inhibition.

Cells are usually present in an organism in the form of a state, which represents a certain state of differentiation (e.g. the functions of the cell are limited to a certain extent as a consequence of specialization of the cells for certain functions, for example a macrophage, a leukocyte, an erythrocyte, a stroma cell, a fat cell a muscle cell, ...). There are also present in an organism certain kind of cells which have the capacity to further differentiate into certain other kinds of cells with other functional properties. Usually differentiation takes place from a less specialized type of cell to a more specialized type of cell. With cells having a differentiation disorder there are meant cells, which differentiate into cells having other functions or properties and which differentiation does not occur normally with this kind of cells. The altered differentiation of these cells with a differentiation disorder can result in more specialized cells or it can result in less specialized cells. Especially cancer cells very often have an altered, not normal differentiation state and often differentiate to less specialized cells or differentiate into a type of cells more typical for embryonic cells or stem cells. Hyperproliferative disorders and differentiation disorders include neoplastic as well as non-neoplastic disorders. Hyperproliferative disorders and differentiation disorders or malfunctions of cells result in disorders such as prostate cancer, breast cancer, lung cancer, colon cancer, rectum cancer, colorectal cancer, anal cancer, ovary cancer, uterus cancer, corpus cancer, cervix cancer, thyroid cancer, bladder cancer, stomach cancer, liver cancer, cancer of blood cells such as non-Hodgkin's lymphoma or other disorders or pre-cancerous or pre-malignant disorders which eventually may convert to cancer such as polyps, warts, cysts, hamartoma, vitiligo, atopic dermatitis, hyperproliferative or UV-responsive dermatoses, psoriasis, actinic keratose, cervix dysplasia, erythroplasia, leukoplakia, lymphomatoid granulomatosis, preleukemia, xeroderma pigmentosum, lymphomatoid papulosis, etc. Also certain virus infections can cause hyperproliferative disorders or differentiation disorders such as avian leukosis, avian sarcoma, Marek disease, pulmonary adenomatousis, viral warts, murine acquired immunodeficiency, eppstain-barr virus infections and papillomavirus infections.

### Disorders

With disorders regarding an organism or regarding an individual are meant all kind of biological changes and diseases resulting in impaired health, reduced life expectancy, or reduced quality of life as a result of changes in biological, physical, mental, psychological or other functions of the organism of an individual. With disorders regarding cells (cells with a hyperproliferative disorder, cells with a differentiation disorder, neoplastic cells, cancer cells, colorectal cancer and related cells, polyp cells, etc.) are meant all kind of biological, biochemical, or other changes resulting in a phenotype of the cell which shows an increased cell proliferation, increased life time of the cell, cells with the property to form metastases, cells with an altered differentiation stage leading to less specialized types of cells such as cells with characteristics of embryonic cells, etc.

### Colorectal cancerous or related disorders

With "colorectal cancerous or related disorders" and "colorectal cancer and related disorders" are meant all kinds of cancer of the colon or rectum, as well as other diseases of the colon and rectum which are associated with colon or rectum cancer or which are associated with cells having a hyperproliferative disorder, cells having a differentiation disorder or neoplastic cells. Among others, all kinds of cancer or cancer-like disorders, including genetic conditions or predispositions, with a likelihood to convert into cancer of the colon or rectum are included in the definition of "colorectal cancerous or related disorders" and "colorectal cancer and related disorders". Among the disorders with a likelihood to convert into cancer the presence of polyps in the colon or rectum is of major importance. A polyp (a clinical term without pathologic significance) is any mass of tissue that arises from the bowel wall and protrudes into the lumen. Polyps may be sessile or pedunculated and vary considerably in size. Such lesions or polyps are classified histologically as tubular adenomas, tubulovillous adenomas (villoglandular polyps), villous (papillary) adenomas (with or without adenocarcinoma), hyperplastic polyps, hamartomas, juvenile polyps, polypoid carcinomas, pseudopolyps, lipomas, leiomyomas, or other rarer tumors (The Merck Manual of Diagnosis and Therapy, 17^{th} edition, 1999, page 326-330). All of these lesions or polyps are, according to the invention, regarded as "colorectal cancerous or related disorders" or as "colorectal cancer and related disorders". Further "colorectal cancerous or related disorders" or "colorectal cancer and related disorders" according to the invention include Lynch syndrome, Gardner's syndrome, Peutz-Jeghers syndrome and Crohn's disease, familial polyposis, chronic ulcerative colitis and granulomatous colitis.

### Xenograft

A xenograft according to the invention comprises at least one cell, originating from a species different to the experimental animal or host, into which the xenograft is implanted. The xenograft can comprise one or more than one cell, a tissue, a tumor, or a whole organ. The xenograft can be a homogeneous population of cells, for example a single clone of a cell line, or it can be a mixture of various types of cells. It can be a mixture of cells with a hyperproliferative and/or a differentiation and/or a neoplastic disorder and it may contain in addition normal, healthy cells not affected by hyperproliferative or differentiation or neoplastic disorders. Especially if tissues, organs or tumors are used as xenograft, these xenografts usually comprise various types of cells for example a tumor often comprises tumor cells of various differentiation stages and in addition healthy normal cells. A clear differentiation between cells with a hyperproliferative disorder, cells with a differentiation disorder and neoplastic cells is often not easy or impossible as there may be smooth transitions from one type of cell to the other or cells may have a differentiation and a hyperproliferative disorder at the same time. It is also possible to transplant two or more different xenografts into a single experimental animal. Preferred kinds of tissues and cells are tumor cell lines, primary tumor cells, tumor tissues or whole tumors from various species, such as mice, rats, monkeys, hamsters , etc., but preferably from mammals, more preferably from humans. Preferably the cells, tissues, organs or tumors are those, which are suitable as model for colorectal carcinomas or related disorders. Numerous of such tumor cell lines are available from suppliers such as the American Tissue Type Collection (ATCC, Manassas, IL, USA), from the European Collection of Cell Cultures (ECACC), Salisbury, United Kingdom, or from the "Deutsche Stammsammlung für Mikroorganismen und Zellkulturen" (DSMZ) (German Collection of Microorganisms and Cell Cultures), Braunschweig, Germany. Primary tumor cells, tumor tissues or whole tumors can be obtained by surgical methods from animals or humans, which developed spontaneously a tumor or can be obtained from experimental animals, which were treated with carcinogenic chemicals or biologicals or which were treated with methods such as radiation, certain virus infections, etc. promoting the appearance of tumor cells.

The xenograft can be implanted into any part of the organism of the experimental animal, including, but not limited to the peritoneal cavity, the brain, the skin, the blood system, organs such as liver, kidney, lungs, the heart, muscles, fat tissue, the skin, the bone marrow, into legs, arms, hands, foot, ears, tails etc. Implantation can be done surgical or by single or repeated injections of cells for example by intravenous, by subcutaneous, by intraperitoneal, etc. injections. Implanting can be done into more than one part of the organism of an individual experimental animal. The size of the xenograft or the number of the cells of the xenograft implanted is at least 1 cell, preferably more than 30, more than 300, more than 3000, more than 30 000, more than 300 000, more than 3 000 000, more than 30 000 000, more than 300 000 000, or preferably more than 3 000 000 000 cells. In general the smaller the experimental animal is, the smaller the number of cells used as xenograft should be. The xenograft transplanted into the experimental animal preferably should be not more than 0.000 000 001 % of the total the cell number of the experimental animal, preferably not more than 0.000 000 01 %, preferably not more than 0.000 000 1 %, preferably not more than 0.000 001 %, preferably not more than 0.000 01 %, preferably not more than 0.000 1 %, preferably not more than 0.001 %, preferably not more than 0.01 %, preferably not more than 0.1 %, preferably not more than 1 % of the total cell number of the experimental animal. If xenografts growing to different sizes are compared, their size (measured as number of cells, volume of tissue, organ or tumor, wet weight of tissue, organ, or tumor) preferably differs at least by 10 %, preferably 20 %, 30 %, 40 %, 50 %, 70 %, 100 %, 150 %, 200 %, 400 %, 600 %, 1000 %, 2000 % or preferably at least by 5000 %. If xenografts growing at different physical positions within a non-human host are compared, these xenografts preferably should grow at least in a distance of 1 %, preferably 2 %, preferably 5 %, preferably 10 %, preferably 20 %, preferably 30 %, preferably at least 50 % of the maximal body length of the host. In case the host is a non-human mammal such as a mouse or rat, the maximal body length of the experimental animal preferably can be measured from the tip of the nose to the end of the tail. If xenografts grown for different periods of time are compared the growth time difference preferably should be more than 1 day, preferably more than 2, more than 3, more than 4, more than 5, more than 6, more than 7, more than 10, more than 14, more than 21, more than 28, more than 1 month, more than 2 months, more than 3 months, more than 4 months, more than 5 months, preferably more than 6 months.

The cells of the xenograft can be manipulated with various methods, prior or during their use as a xenograft. Growing of the xenograft in vitro or in immune competent or immune-compromised experimental animals can be done to simply proliferate the cells of the xenograft, or to change the differentiation grade of the cells of the xenograft. Alternatively the cells of the xenograft can be treated with substances such as chemotherapeutics, growth factors, hormones or infected with viruses, or subjected to environmental factors such as radiation with the intent to purify or enrich the cells of the xenograft for cells with certain characteristics. These characteristics among other can be a faster or slower growth rate of the cells, selection for cells with genetic mutations or with increased or decreased mutation rates, selection for cells with changes in their differentiation grade, selection for cells with a higher or lower tendency to form metastases, or selection for cells with a tendency to preferably grow as metastases or to preferably grow as metastases in certain organs or tissues, selection for cells with higher or lower viability which are more resistant or less resistant to certain anti-cancer treatments, such as cancer radiation therapy, cancer surgery, cancer chemotherapy, cancer hormone therapy, etc.

The cells of the xenograft can be manipulated for example by repeatedly transplanting, growing and re-transplanting them into another experimental animal. They can be manipulated by radioactive irradiation such as gamma radiation, neutron radiation, electron radiation or proton radiation. Radiation can be done by external beam radiation or by use of radioactive substances directly applied to the cells or the experimental animal. The cells of the xenograft can be treated in vitro or in vivo with substances and/or peptides and/or proteins and/or nucleic acids, such as antibiotics, cancer chemotherapeutics or other medicaments, growth factors, angiogenesis factors, factors with promote or which inhibit cell division and/or differentiation, substances which promote or which inhibit genetic mutations, substances which enable to selectively enrich certain subpopulations of cells of the xenograft for example cells resistant to cancer chemotherapy, etc. For example tumor cells can be injected into experimental animals and the animals can be treated with anti cancer drugs and subsequently the remaining, now more drug resistant tumor cells can be isolated from the experimental animal and used in another experimental animal to identify markers of drug-resistant tumor cells, using the methods described in the invention.

The substances, which can be used and which have various effects on the cells of the xenograft include, but are not limited to small organic or inorganic molecules such as anti-cancer drugs, peptides, proteins and nucleic acids, for example nucleic acids coding for drug resistance genes, coding for oncogenes, coding for angiogenetic or anti-angiogenetic factors, coding for tumor suppressor genes, vectors or viruses which for example increase the mutation rate or which result in immortalization of the cells, or which induce carcinogenesis, etc. or nucleic acid molecules such as antisense, ribozymes, RNAi, etc. which can modify the expression of oncogenes or tumor suppressor genes. Examples of suitable substances among others are alkylating drugs such as mechlorethamine, chlorambucil, cyclophosphamide, melphalan or ifosfamide, antimetabolites such as methotrexate, 6-mercaptopurine, 5-fluorouracil, cytarabine, gemcitabine, vinblastine, vincristine, vinorelbine, paclitaxol, docetaxel, etoposide, irinotecan or topotecan, antibiotics such as doxorubicin, bleomycin or mitomycin, nitrosoureas such as carmustine or lomustine, inorganic ions such as cisplatin or carboplatin, biologic response modifiers such as interferons, interleukins, interleukin-2 or tumor necrosis factor (TNF), enzymes such as asparaginase or hormones such as tamoxifen, leuprolide, flutamide or megestrol acetate, viruses or vectors such as, eppstain-barr virus, papillomavirus, or other tumorigenic viruses, etc.

### Samples, negative control samples, reference values and comparisons of samples

According to the invention any sample comprising peptides and/or proteins is suitable as a sample. Preferably the sample is whole blood, blood cells (such as leukocytes, B-cells, T-cells, monocytes, macrophages, erythrocytes and thrombocytes), serum, plasma, hemofiltrate, liquor cerebrospinalis, urine, lymph, lymph knot tissue, tumor tissue or tumor-related tissue such as tissue of polyps or other tissues and organs as well as combinations thereof. Preferably the host is a mammal, a primate, a rodent, a human, a mouse or a rat. Preferably the sample comprises at least 10, preferably at least 50, preferably at least 100, preferably at least 200, preferably at least 600, and preferably at least 1200 different peptides and/or proteins. Preferably a plurality of peptides and/or proteins are determined from a sample which means that preferably at least 10, preferably at least 50, preferably at least 100 preferably at least 200, preferably at least 600 and preferably at least 1200 peptides and/or proteins are determined. With determined is meant, that these peptides and/or proteins are analyzed qualitatively and/or quantitatively. Subsequently differences to the same peptides and/or proteins present or absent in negative control samples or differences to reference values are identified (in the following also referred to as altered quantities of the peptide). Samples to be compared among others can be samples originating from a host comprising a xenograft compared with samples from a host without a xenograft or with a different xenograft . In another embodiment, samples from a host with a xenograft grown for different periods of time are compared, alternatively, samples from a host with a xenograft grown at different physical positions within the organism of the host, or samples from hosts with xenografts grown to different sizes. In addition, samples from hosts with a xenograft may be compared with samples from hosts which have been treated with a therapy to remove the xenograft or to kill the cells of the xenograft or to suppress the growth or viability of the cells of the xenograft In another aspect, samples from hosts with differently treated xenografts are compared with each other, or plasma samples from a host comprising a xenograft may be compared with urine samples from the same or a different host comprising the same xenograft or comparisons of other combinations of samples. The samples from one time point / from one physical position / from one xenograft size / from a xenograft treated with one therapy / from one kind of sample source, for example plasma / etc. are the samples of the individual to be tested and the samples from another time point / from another physical position / from another xenograft size / from a xenograft treated with another therapy or from a xenograft not treated with a therapy / from another kind of sample source, for example urine / etc. are the negative control sample. Possible factors resulting in differences between xenografts can be the differentiation stage of the cells of the xenograft, the presence or absence of certain oncogenes, tumor suppressor genes, transcription factors, receptors for growth hormones, receptors for angiogenetic factors, receptors for chemokines, receptors for cytokines, adhesion molecules at the cell surface, binding partners for adhesion molecules, secreted chemokines, cytokines, hormones, proteases, angiogenetic or anti-angiogenetic factors, etc. The results of all of these comparisons enable in subsequent analysis of samples of hosts with a xenograft of unknown identity to judge, whether the unknown xenograft will grow rapidly or slowly, will have a tendency to grow in localizations of the body distant from the localization where it was implanted originally (for example metastases) or what kind of oncogenes, tumor suppressor genes, receptors and ligands a xenograft comprises. Finally this knowledge can be extended to for example human patients with unknown cells with a hyperproliferative disorder, and/or cells with a differentiation disorder and/or cells with a neoplastic disorder. This information can be used to diagnose the presence and the properties of said cells within the individual or patient or host and to precisely adjust a therapy to the specific type and properties of the cells, present in the organism of the individual or patient. This would also allow to predict, which treatment an individual or patient should get or would allow to monitor an individual or patient for a possible relapse of a disorder caused by said cells.

With quantity-changes of peptides and/or proteins according to the invention is meant that the amount of the peptides differs between the sample and the negative control sample. The term "amount" according to the invention means the presence or absence of a substance or the determined quantitative or semi quantitative relative or absolute concentration or quantity of a substance. Preferably the amount of the substance between the sample and the negative control or the reference value is altered for example is differing by at least 5 %, preferably 10 %, preferably 20 %, preferably 40 %, preferably 60 %, preferably 100 %, preferably at least is differing 2-fold, preferably at least 5-fold, preferably 10-fold, preferably 20-fold, preferably is differing at least 50-fold. Differences between the sample and the negative control sample are always calculated relative to the lower of the two values. It is also possible, that either in the sample or in the negative control sample the peptide and/or protein is not detectable, or is below the detection limit of the method used for detection.

A reference value according to the present invention can e.g. be determined empirically. For this purpose, the concentration of the peptide(s) is measured in a series of samples from individuals having a defined status with respect to the presence or absence of cells of the group consisting of cells having a hyperproliferative disorder, cells having a differentiation disorder, and neoplastic cells.

Properties of cells with a hyperproliferative disorder and/or differentiation disorder and/or neoplastic disorder can be correlated with the quantity-changes of the markers identified with the method of the invention. In general the correlation between the marker and the properties of said cells can be a negative or a positive correlation. For example, if a certain marker rises in a patient sample this could indicate that neoplastic cells are growing as metastases in said patient (positive correlation of the marker and metastasis activity) and if a certain other marker rises in a patient sample this could indicate, that the neoplastic cells present in said patient are not growing as metastases (negative correlation of marker and metastasis activity). It is also possible, that panels of more than one maker are needed to predict the properties of said cells, for example if one marker rises in quantity and a second marker rises in quantity and a third marker decreases in quantity the neoplastic cells present in said patient are not growing as metastases. Preferably more than 20, preferably up to 20, preferably up to 10, preferably up to 7, preferably up to 5, preferably up to 4, preferably up to 3, preferably up to 2 preferably only 1 different markers are needed to predict a certain property of said cells or to predict several certain properties of said cells. With different markers are meant all kind of peptides differing in their sequence or in modifications of their sequences, such as posttranslational modifications, regardless if these peptides originate from the sequence of the same or of different protein or peptide sequences.

### Peptides and proteins

The term "peptide" refers to compounds comprising two or more, preferably 3 or more, 4 or more, 6 or more, 8 or more, 10 or more, 13 or more, 16 more, preferably 21 or more amino acids joined covalently by peptide bonds. Big peptides are commonly termed proteins but in general peptides and proteins are synonyms. The term "TIF 1-beta" is used herein without the addition of the word peptide or fragment, for example TIF1-beta and not TIF1-beta peptides or TIF1-beta and not TIF1-beta fragments, for the naturally occurring protein. That means, TIF1-beta represents the complete amino acid sequence of TIF 1-beta with or without the N-terminal Methionine, which might be removed due to processing of TIF1-beta . TIF1-beta fragment is a synonym for TIF1-beta peptide. Preferably the peptides and/or proteins of the invention are purified to at least 30 %, preferably 40 %, preferably 50%, preferably 60 %, preferably 70 %, preferably 75 %, preferably 80 %, preferably 85 %, preferably 90 %, preferably 95 %, preferably 97 %, preferably 99 %, preferably more than 99 % purity. The peptide and/or proteins of the invention can comprise mixtures of similar peptides and/or proteins such as derivatives and/or polymorphic forms of the sample peptide and/or protein, for example a non-glycosylated form of a certain peptide or protein and one or several differently glycosylated forms of said peptide. It is common, that certain modifications such as glycosylations, phosphorylations, etc. are not present in all molecules of an otherwise homogeneous peptide or protein sample, as in nature not all molecules are subjected to these modifications or as some modifications take place only partially or are reversible or as there exists an equilibrium between different derivatives of a certain peptide or protein.

The terms "peptide" and "protein" according to the invention include compounds comprising only amino acids, as well as compounds also comprising non-amino acid constituents such as carbohydrates, lipids, phosphor groups, etc. and include compounds comprising only peptide bonds and compounds also comprising other bonds, e.g. ester, thioester or disulfide bonds.

### Modifications of peptides

Modifications of peptides or proteins according to the invention may comprise modifications due to posttranslational modifications, chemical modifications, enzymatic modifications and modifications due to other mechanisms. Examples of possible modifications include but are not limited to: glycosylation, phosphorylation, sulphatylation, pyroglutamate modification, cystein-disulfide bridges, thioester bridges, methylation, acetylation, acylation, farnesylation, formylation, geranylgeranylation, biotinylation, stearoylation, palmitylation, lipolyation, C-mannosylation, myristoylation, amidation, deamidation, methylation, demethylation, carboxylation, hydroxylation, iodination, oxidation, pegylation, prenylation, ADP-ribosylation, addition of lipids, of phosphatidylinositol, of glycosylphosphatidylinositol (GPI)-anchor, of pyridoxal phosphate, modification of cystein residues resulting in carboxyamidomethylcysteine, resulting in carboxymethylcysteine, or resulting in pyridylethylcysteine, modification of lysine residues resulting in liponic acid, modification of glutamic acid resulting in pyroglutamic acid, etc.

Modifications of peptides or proteins according to the invention may comprise unusual amino acids, chemically or enzymatically modified amino acids etc. including, but not limited to: alpha amino butyric acid, beta amino butyric acid, beta amino iso-butyric acid, beta alanine, gamma butyric acid, alpha amino adipic acid, 4-amino benzoic acid, amino ethyl cysteine, alpha amino penicillanic acid, allysine, 4-carboxy glutamic acid, cystathionine, carboxy glutamic acid, carboxy amido methyl cysteine, carboxy methyl cysteine, cystein acid, citrulline, dehydroalanine, di-amino butyric acid, dehydro amino-2-butyric acid, ethionine, glycine-proline di-peptide, 4-hydroxyproline, hydroxylysine, hydroxyproline, homoserine, homo cysteine, histamine, iso-valeine, lysinoalanine, lanthionine, norvaline, norleucine, ornithine, 2-pipiridine-carboxylic acid, pyroglutamic acid, pyrrolysine, proline-hydroxy proline di-peptide, sarcosine, 4-selenocysteine, syndesine, thioproline, etc. Further examples can be found in databases such as the "Delta Mass" database searchable at the website of the ABRF, the "Association of Biomolecular Resource Facilities": http://www.abrf.org/index.cfm/dm.home?AvgMass=all

### Derivatives, polymorphic forms and fragments of peptides

Derivatives of peptides or proteins are primary characterized by modifications of one or more amino acid structures or modifications of the peptide bond structure, whereas polymorphic forms of peptides or proteins are characterized by differences in the amino acid sequence. It is possible, that a peptide or protein at the same time is a derivative and a polymorphic form of another peptide or protein.

With derivatives of peptides and/or proteins are meant all kind of peptides and/or proteins and/or fragments thereof, including peptides and/or proteins comprising posttranslational modifications, chemical modifications, enzymatic modifications and modifications due to other mechanisms. Derivatives of peptides may comprise amino acid residues different from the standard set of 20 amino acids and/or may comprise peptidomimetic structures.

With polymorphic forms of peptides and/or proteins are meant variations of the sequences due to mutations present in nature or due to mutations caused by experimental or random manipulation of the sequence using techniques such as molecular biology, genetics or chemistry (nucleic acid or peptide synthesis).

Furthermore polymorphic forms of peptides and/or proteins preferably have 70 % sequence identity, preferably 75 %, preferably 80 %, preferably 85 %, preferably 90 %, preferably 95 %, preferably 97 % and preferably have 99 % sequence identity with each others as determined by sequence alignment. An amino acid residue is regarded as identical in two sequences, if within a sequence alignment of these two sequences this amino acid residue is placed at the same position. If for example a first hypothetical sequence "ACDEFGHIKL" and a second hypothetical sequence "ACDEFHIKL" with a deleted "G" are aligned, there would be inserted a gap into the second sequence between "F" and "H" resulting in "ACDEF-HIKL" thereby enabling a better alignment for of the second sequence to the first sequence. Consequently, in the resulting alignment there are now 9 out of 10 positions occupied by identical amino acid residues resulting in a sequence identity of the first to the second sequence of 90 %. Methods how to calculate sequence alignments are given below.

Preferably a peptide or protein which represents a fragment of TIF1-beta, and/or a derivative and/or a polymorphic form at least comprises 8 amino acid residues, preferably 10, preferably 12, preferably 15, preferably 20, preferably 50, preferably 100, preferably 200, preferably 300, preferably 400, preferably 500, preferably 600, preferably 700, preferably at least 800 amino acid residues, which are present at identical positions within the alignment of the peptide or protein and TIF1-beta.

### Precursors of the TIF 1-beta fragment

"Precursors of TIF 1-beta fragment" according to the invention can comprise the complete sequence of TIF 1-beta (Seq.-ID 6 and 7) or a fragment of TIF 1-beta. In addition, "precursors of TIF 1-beta fragment" according to the present invention can also be derivatives and/or polymorphic forms of TIF 1-beta or TIF 1-beta fragments. "Precursors of a TIF 1-beta fragment" are processed or modified resulting in TIF 1-beta fragments, thus, TIF 1-beta fragments represent peptides which are structurally different to the precursor of TIF 1-beta fragments. The processing or modification of the precursor may be the result of any kind of enzymatic, chemical, mechanical, or other type of reaction. Examples of such reactions converting a precursor of a TIF 1-beta fragment into a TIF 1-beta fragment among others are proteolytic digestion, dephosphorylation, glycosylation, de-glycosylation, formation or destruction of disulfide bonds, oxidation, reduction, etc. Generally, all kind of processing and/or modification of the precursor are possible which e.g. may take place in vivo in the individual, or in vitro, for example in the sample or in the negative control sample during storage.

### Preparing of sequence alignments and calculation of sequence homology

To calculate the homology of sequences computer programs such as the GCG software suite (Genetics Computer Group, University of Wisconsin, Madison, WI, USA), including GAP (Nucleic Acids Res. 12:387-95), BLASTP, BLASTN, FASTA (J Mol Biol. 215:403-10) or the well known Smith Watermann-algorithm can be used. Preferred parameter used for amino acid comparisons or alignments comprise the algorithm of Needleman and Wunsch (J Mol Biol. 48:443-53), the BLOSUM 62 matrix (Proc Natl Acad Sci USA. 89:10915-9), a gap penalty of 12, a gap length penalty of 4 and a threshold of similarity of 0. The GAP software is also suitable to be used with the parameters mentioned. The stated parameters are the default parameter for amino acid comparisons, wherein gaps at the end of a sequence do not decrease the homology value. If very short sequences such as 8 to 20 amino acids long sequences are compared it may be necessary to increase the expectation value up to 100 000 and to reduce the word length down to 2. Further suitable algorithms are the use of gap opening penalties, gap extension penalties and the use of matrixes found in the program handbook, Wisconsin Package, version 9, from September 1997. The choice of the most suitable algorithms depends on the kind of comparisons to perform. If two sequences are compared the GAP or the Best Fit algorithms are preferred, if one sequence is compared to a sequence database the FASTA and BLAST algorithms are preferred. A sequence identity of 70 % is also termed a homology of 70 %. Identity means, that at the same position within both sequences of the alignment the same amino acid residue is present.

### Peptidomimetics

The peptides listed in table 1, preferably TIF1-beta and/or TIF1-beta fragments according to the invention can be prepared or synthesized to represent partially or completely peptidomimetics. This allows to design peptides and/or proteins with more defined properties. For example D-amino acids could be used to prevent proteolysis of peptide bonds. "Peptidomimetics" according to the invention are structures, which mimic the function of amino acids or amino acid sequences. "Peptidomimetics" or "mimetics of peptides" often have additional properties such as increased resistance to proteolysis. Peptidomimetics can replace some or all peptide bonds by other kinds of covalent bonds which can connect amino acids. Peptidomimetics among others can comprise (i) amino acid modifications, (ii) di-peptide analogues, (iii) peptide backbone modifications which for example increase the biological half-life and (iv) secondary structure mimics. Some peptidomimetics for example limit the degree of freedom to the peptide. Examples of amino acid modifications are alpha-C alkylation, alpha-N alkylation, etc., di-peptide analogues peptides with two amino acid side chains connected to each other by covalent bonds, or modifications of the peptide backbone, such as amide bond isosteres or retro-inverso isomers obtained by changing from L- to D-amino acids and inverse N- to C-sequence, etc. Secondary structure mimetics comprise mimetics of structures such as alpha-helices (for example by beta-amino acids), beta-sheets, beta-turns (for example by linchpin structures) and gamma-turns. Further examples of peptidomimetics are spiegelmers® (NOXXON Parma AG, Berlin, Germany) or modifications such as pegylation, which can modify the solubility or immunogenic properties of peptides and proteins.

### Nucleic acids, complementary and degenerated nucleic acids and derivatives

With nucleic acids or nucleic acid sequences according to the invention are meant all kinds of deoxyribonucleic acids (DNA) and ribonucleic acids (RNA) or combinations thereof regardless if these nucleic acid molecules are single or double-stranded, linear, circular or branched. Preferably the nucleic acid molecules are purified nucleic acid molecules which means that the preparation contains at least 50 %, preferably at least 60 %, preferably at least 70 %, preferably at least 80 %, preferably at least 90 %, preferably at least 95 %, preferably at least 99 %, preferably more than 99 % of a certain nucleic acid molecule or of a certain mixture of more than one certain nucleic acid molecule. Examples of nucleic acid molecules are genomic DNA, cDNA (complementary DNA), mRNA (messenger RNA), recombinantly produced nucleic acid molecules, chemically synthesized nucleic acid molecules or enzymatically generated nucleic acid molecules for example by use of polymerase chain reaction (PCR) or nucleic acid molecules purified from natural sources such as prokaryotic or eukaryotic cells, viruses, tissues, organs or whole organisms such as bacteria, yeast, insects, worms, etc. using methods known in the art. Furthermore nucleic acid molecules according to the invention can be partially or completely represent derivatives of nucleic acids which derivatives comprise nucleotides or nucleotide like molecules not found in nature such as phosphorothioates, peptide nucleic acids (PNAs), N3',P5'-phosphoramidates, morpholino phosphoroamidates, 2'-O-methoxyethyl nucleic acids, 2'-fluoro-nucleic acids, arabino-nucleic acids, locked nucleic acids (LNA, ribonucleotides containing a methylene bridge that connects the 2'-oxygen of ribose with the 4'-carbon).

Nucleic acids according to the invention furthermore comprise antisense nucleic acids, ribozymes, triplex-forming nucleic acids, RNAi-nucleic acids, nucleic acids suitable as primer for polymerase chain reactions, suitable for in situ hybridization, suitable for hybridization experiments such as Northern or Southern-blots or suitable for nucleic acid chip experiments, nucleic acids suitable as vectors or viral vectors, preferably for expression of a molecule listed in table 1, preferably TIF1-beta and/or TIF1-beta fragments or for expression of antisense nucleic acids specific for TIF1-beta and/or TIF1-beta fragments. The nuclei acid molecules preferably have a length of the full length of the mRNA coding for a molecule listed in table 1, such as TIF1-beta mRNA, preferably less than 600, 500, 400, 300, 200, 100, 50, 40, 30, 25, 20, 15 nucleotides, depending on the intended use. If the nucleic acid represents an expression vector, the length of the nucleic acid molecule can be much longer up to several thousands of nucleotides as many viruses such as for example bacculoviruses have large genomes resulting in very large vectors. Antisense and triplex-forming nucleic acid molecules preferably have a sequence length of 6 to 50, preferably 10 to 30, 15 to 30, 20 to 30 nucleotides. Ribozymes preferably have sequences hybridizing to the target RNA, which hybridizing sequences have similar length as corresponding antisense nucleic acids and in addition comprise a catalytic ribozyme sequence as known in the art. Nucleic acids for use as primer in polymerase chain reactions preferably have a length of 10 to 60, preferably 15 to 60, 15 to 50, 15 to 40, 15 to 30 nucleotides.

With complementary nucleic acid sequences are meant nucleic acid sequences which bind to each other due to interaction of Guanine with Cytosine and Adenine with Thymidine/Uracil. Binding of complementary nucleic acids to each other is called hybridization. The hybridization preferably takes place under stringent experimental conditions which ensure that the hybridization is sequence specific and not random. Stringent hybridizations conditions for nucleic acids are known in the art, and are published for example in Molecular Cloning: A Laboratory Manual, J. Sambrook et al, Cold Spring Harbor, New York, 1998. An example for stringent conditions for hybridization conditions is the hybridization at 65 °C in a buffer containing 3.5x SSC, 0.02 % Vicoll, 0.02 % Polyvinylpyrrolidon, 0.02 % bovine serum albumin, 2.5 mM NaH₂PO₄ (pH 7), 0.5 % SDS (sodium dodecyl sulphate), 2 mM EDTA (Ethylenediaminetetraacetic acid ). SSC represents a buffer containing 0.15 M sodium chloride and 0,15 M sodium citrate at pH 7. Subsequently to hybridization the membrane with the hybridized nucleic acid sticking to it is washed at room temperature with 2x SSC and with 0.1 to 0.5x SSC with 0.1 % SDS at temperatures up to 68 °C. It is not necessary that the complete nucleic acid sequences hybridize to each other, as long as the hybridizating nucleic acid structure is stable under stringent experimental conditions. A specific hybridization requires that at least 60 %, preferably at least 70 %, 80 %, 90 %, 95 %, 99 % of the sequence sections of the nucleic acid sequences hybridizing to each other are complementary to each other Nucleic acids can also be detected by hybridization of nucleic acid sequences 5' to the start codon or 3' to the stop codon of an mRNA or a cDNA, as long as these parts of the mRNA or cDNA are specific for the nucleic acid sequence.

With degenerated nucleic acid sequences is meant that a distinct amino acid sequence can be encoded for by various nucleic acid sequences, as most amino acids are encoded by at least two different codons. For this reason identical amino acid sequence can be encoded by very different sequences of nucleic acid sequences.

### Labeled nucleic acids, peptides, proteins and binding agents

The term "label", as used herein, refers to any moiety that functions to: (i) provide a detectable signal; (ii) interact with a second label to modify the detectable signal provided by the first or second label, e.g. FRET; (iii) affect mobility, e.g. electrophoretic mobility, by charge, hydrophobicity, shape, or other physical parameters, or (iv) provide a capture moiety, e.g., affinity, antibody/antigen, or ionic complexation, resulting in degenerated nucleic acid sequences.

The invention comprises substances such as nucleic acids, peptides, binding agents, antibodies as well as fragments and derivatives of said substances, which can comprise a label or other functional group. Suitable are all kinds of labels and functional groups, such as fluorescent molecules, radioactive molecules, luminescent molecules, enzymes, toxins, dyes, metal particles, magnetic particles, polymer particles, biotin or other organic molecules. Especially for mass spectrometry isotope labels or isobaric labels are suitable. Isotope labels can be obtained by use of isotopes with molecular weights different to their natural molecular weight such as oxygen-18, nitrogen-15, deuterium, tritium, carbon-14, sulfur-35, or phosphorus-32 etc. Isobaric labels have the same molecular weight, as long as these labels are intact and have not been fragmented in a mass spectrometer. Therefore isobar labels can more conveniently be analyzed using mass spectrometric methods, as only a limited number of mass differences is present prior to analysis of the sample. Examples of fluorescent molecules are fluorophores such as fluorescein, Texas red, rhodamine, BODIPY, cyber-green, etc, fluorescent proteins or fragments or derivatives thereof such as green fluorescent protein, red fluorescent protein, blue fluorescent protein, renilla fluorescent protein, etc. Examples of radioactive isotopes suitable as label or functional group are phosphorous-32 or -33, sulphur-35, iodine-125, tritium, carbon-14, calcium-45, chromium-51 and all other known radioisotopes which can be coupled to nucleic acids and/or peptides and/or proteins. Examples of enzymes are horse reddish-peroxidase, alkaline phosphatase, luciferase, galactosidase, etc. Suitable toxins are all kinds of toxins such as microbial toxins, toxic peptides or toxic small organic molecules, synthetic toxins or toxins used for medical purposes. An example of a suitable dye is Digoxigenin. Examples of particles are of various particle diameters, preferably between 0.1 to 100 µm. Metal particles are particles can be made from various substances, preferably heavy elements such as gold, silver, platinum or other suitable metals or alloys or mixtures thereof. Examples of magnetic particles are particles preferably made from all kind of magnetic or magnetize able materials including iron, preferably iron oxides such as Fe₃O, Fe₂O₃, Fe₃O₄, etc. Examples of polymer particles are particles made from a polymer such as polyacrylamide, agarose, polypropylene, polystyrene, polytetrafluoroethylene, etc. Various other labels or functional groups, such as biotin, chitin, maltose or glutathione etc. as known in the art, can also be used.

Furthermore labels or functional groups comprising certain additional nucleic acid or amino acid sequences subsequently termed label-sequences can be used. Label-sequences comprising nucleic acid sequences for example can be used for detection of the labeled substance for example by hybridization with a probe specific for the label-sequence or by detection of the label-sequence by polymerase chain reaction, etc. Examples of label-sequences comprising amino acid sequences are his-tags, flag-tags, myc-tags or whole proteins or fragments thereof such as antibodies, glutathione S-transferase, streptavidin, chitin-binding protein, maltose binding protein, various lectins, etc. These labels can be detected by use of antibodies or by use of ligands binding to these labels such as protein A, protein G, protein Y, protein A/G, glutathione, biotin, chitin, maltose or other sugars or these label sequences can function in other ways such as the transport of the labeled substance into the cell, if the label-sequence is for example a HIV-Tat peptide, Antennapedia peptide, VP22 peptide, etc. Label-sequences in general can also be used to purify the label substance by capturing the labeled substance by a binding agent specific for said label-sequence, especially if the label-sequence comprises a amino acid sequence.

### Vectors

Nucleic acid sequences can be part of a vector. A vector according to the invention can be circular or linear or branched, single or double stranded, can be composed of ribonucleic acids, deoxyribonucleic acids or combinations thereof. The vector can be a naked nucleic acid, a nucleic acid associated with for example proteins, lipids, liposomes, calcium-phosphate precipitates or other substances or combinations thereof. The vector can also be a virus or cell comprising said nucleic acids or vectors. The vector can be suitable to enhance or decrease the amount of for example TIF1-beta and/or of TIF1-beta fragments and/or derivatives or polymorphic forms thereof or of other molecules listed in table 1. Vectors increasing the amount of said peptides and/or proteins, fragments or derivatives thereof are for example transient or stable expression vectors. Vectors decreasing the amount of said peptides and/or proteins, fragments or derivatives thereof for example comprise nucleic acid sequences coding for example for TIF1-beta sequence-specific antisense-nucleic acids, ribozymes, triplex-forming nuclei acids, RNAi-molecules, etc. The vector can be present in the form of a virus such as a retrovirus, an adenovirus, a Bacculovirus, a phage or other viruses known in the art. The virus can be a viable virus or a virus needing a helper-virus for proliferation and/or infection. The vector can be unspecific or specific for certain species such as humans, mice, rats, etc. or groups of species such as mammals, rodents, etc., or certain organs or tissues such as muscle-tissue, pancreas, liver, fat-tissue, etc. certain types of cells such as fat cells, muscle cells, cells of the pancreas, neuronal cells, cells from the gastrointestinal tract, etc. or specific for bacteria, yeasts, insect cells, cell lines such as COS-cells or CHO-cells. The specificity of the vector can be due to specific promoter-nucleic acid sequences which are only active in specific cell types or species, or the vector can be specific by mechanisms targeting the vector to specific cells, or a promoter present in the vector can be activated or can be inactivated by substances given to the individual or cell comprising said vector, etc.

### Host cells

"Host cell" does not mean a cell from the host of a xenograft, but host cells means cells comprising a nucleic acid and/or a vector as described in the previous chapters. The nucleic acid or vector can be present in transient or in stable form in the cell and the nucleic acid or vector can be present in the cytosol or certain organelles of the cell such as the nucleus or mitochondria. The vector sequence or fragments thereof can be present in the cytosol for example in the form of a plasmid or the vector sequence can be partially or completely integrated into the genome of the cell or the genome of the mitochondria, or into a plasmid present in the cell by site (sequence) specific, by random or by other mechanisms.

### Binding agents, antibodies and derivatives

Another embodiment of the invention are binding agents specific for TIF1-beta-, PTA-, or CK18-proteins or binding agents specific for the proteins from which the peptides with the molecular masses of 2315 or 2916 Dalton (both identified in fraction 35), as shown in Table 1, originate. In addition the invention comprises binding agents specific for a fragment, or polymorphic form or derivative of the mentioned peptides or proteins. In particular, the binding agents according to the present invention specifically bind to neoepitopes of a peptide according to any one of Seq.-ID 1 to 5 with the proviso that they do not bind to a peptide according to Seq.-ID 6 or 7. Preferably these binding agents are specific for human proteins or peptides. Preferably the binding agents are specific for a peptide listed in Table 1 or a fragment or polymorphic form thereof. Binding agents according to the invention are among others receptors specific for these peptides, phages binding specific to these peptides, spiegelmers® binding specific to these peptides, small organic molecules binding specifically to these peptides, particles or surfaces coated with substances specifically binding to these peptides, such as receptors, phages, small organic molecules, spiegelmers®. Preferably specific binding agents comprise specific antibodies binding said peptides and/or proteins or fragments or derivatives or polymorphic forms thereof. Also included are fragments of said antibodies as long as these fragments specifically bind to said peptides and/or to said proteins or to said fragments or to said derivatives or to said polymorphic forms. Preferably the binding agent specific for said peptides and/or proteins or fragments or derivatives thereof is an antibody such as an IgG-, IgA-, IgE-, IgD- or an IgY-antibody originating from any species in purified form, or present for example in the form of a cell culture supernatant, an antiserum, as ascites fluid or contained within eggs. The antibodies preferably can originate from humans, mice, rats or other mammals or from eggs preferably from chicken eggs or the antibodies can be produced using recombinant techniques know in the art. The antibody can be a monoclonal, an oligoclonal or a polyclonal antibody. The antibody preferably can be a purified specific antibody, a purified specific antibody further comprising non-specific other antibodies or other proteins such as albumin, an antibody prepared from whole anti-serum, prepared from cell culture supernatant, prepared from eggs or prepared from ascites fluid or an unpurified antibody, preferably unpurified anti-serum, unpurified cell culture supernatant, unpurified ascites fluid or an unpurified recombinant antibody. Preferably a purified antibody or antibody fragment or derivative according to the invention comprises at least 30 %, preferably at least 40 %, at least 50 %, 60 %, 70 %, 75 %, 80 %, 85 %, 90 %, 95 %, or preferably at least comprises 98 % of said antibodies and/or antibody fragments and/or derivatives thereof.

With specific binding agents are meant for example antibodies, which bind specifically to a protein or peptide ligand such as a TIF1-beta or a TIF1-beta fragment, or are meant nucleic acids, which binds specifically to a nucleic acid of the invention. The specificity of the binding of an binding agent such as an antibody or an nucleic acid for example can be tested by standard western blotting, northern blotting, reverse transcriptase-polymerase chain reaction (RT-PCR) or other methods known in the art. As samples there can be used for example cells transfected with a nucleic acid coding for a TIF1-beta fragment for which the antibody to be tested is supposedly specific. As negative control the same but not transfected cell line or a cell line transfected with a non-TIF1-beta fragment can be used. For determining the specificity of antibodies cell lysates from both cell lines are tested in a western blot. A specific binding agent, such as an antibody, recognizes a band of the correct molecular weight only in the lysate from the transfected cells but not in the lysate from the negative control. For determining the specificity of nucleic acids, RNA can be purified from both cell lines and tested in for example northern blots. A specific binding nucleic acid recognizes a band of the correct molecular weight only in the RNA sample of the transfected cell line but not in the RNA from the negative control, resulting in a signal of the appropriate molecular weight on the northern blot. However, as known in the art, there can also be signals on the northern blot in the negative control, but the signal in the negative control is clearly lower, if the binding agent is specific for the ligand transfected into the cell line. The specificity can be further tested by increasing the stringency of the washing steps in the northern blots, which should gradually reduce the signal in the negative control, whereas the signal of the TIF1-beta transfected cells remains present.

The invention furthermore comprises derivatives of antibodies such as an antibody comprising parts of antibodies originating from different species, for example humanized antibodies, which for example comprise the antigen binding domain of a murine antibody and other parts of the antibody or antibody fragment originate from human antibodies. The invention also includes fragments and derivatives of antibodies as long as these antibodies still specifically bind to TIF1-beta fragments and/or derivatives or polymorphic forms thereof, or to other peptides or proteins listed in table 1. Examples of such antibody fragments are Fab2- or Fab antibodies. Further derivatives of antibodies according to the invention are antibodies or fragments of antibodies covalently or non-covalently bound to other substances such as organic compounds, inorganic compounds, peptides or proteins. Examples for such derivatives of antibodies are antibodies labeled with radionuclides, toxins, enzymes, fluorophores, chromophores, biotin or other organic compounds, fluorescent proteins, dyes, or label sequences such as the label sequences described in previous chapters.

### Pharmaceutical compositions

Another embodiment of the invention are pharmaceutical compositions. These pharmaceutical compositions comprise substances such as the peptides listed in table 1 and/or the corresponding complete protein sequences or fragments or derivatives or polymorphic forms of said peptides and/or proteins, nucleic acids coding for said peptides and/or proteins, or fragments or derivatives of said nucleic acids, vectors comprising said nucleic acids, host cells comprising said nucleic acids or said vectors, binding agents or antibodies specific for said peptides and/or proteins or fragments or derivatives of said binding agents or antibodies.

The pharmaceutical compositions of the invention can be used for therapy, prophylaxis, treatment of a relapse of a disorder, diagnosis and prognosis, preferably of disorders caused by cells with a hyperproliferative disorder and/or a differentiation disorder and/or a neoplastic disorder. Preferably the pharmaceutical composition is used for diagnosis, prophylaxis or treatment of cancer cells originating from various types of cancer as described in previous chapters, preferably from cells from colorectal cancerous or related disorders.

The pharmaceutical composition according to the invention can be a medicine or medicament for use in therapy and/or for prevention and/or for treatment of a relapse and/or for prophylaxis of a disorder caused by cells with a hyperproliferative disorder and/or a differentiation disorder and/or a neoplastic disorder. The pharmaceutical composition can be used for individuals suffering from cancer, preferably suffering from colorectal cancerous or related disorders and it can be suitable as a diagnostic tool to determine, if a said disorder is present or soon will be present in the individual tested.

The pharmaceutical compositions can be administrated orally, sublingually, injected intra venous, injected subcutaneously, injected into a tumor, into muscle, fat or other tissues, administered topical onto the skin or onto a mucosa, inhaled as aerosol into the lungs or administrated rectal or through other routes. The pharmaceutical composition can be supplied as tablets, pills, capsules, powders, granulates, salves, plasters releasing substances transdermal to the individual, tinctures, uvula, suspensions, solutions, which solutions preferably are dissolved in sterile physiological sodium chloride solutions for use in injections or as continuous infusions, etc. The pharmaceutical compositions can comprise additives such as filling additives, antimicrobial additives, preservative additives, coloring additives, flavoring additives, smelling additives, protective or stabilizing additives, etc. Preferred protective or stabilizing additives for example are substances which inhibit degradation of peptides and proteins for example protease inhibitors or carrier proteins such as albumins, preferably human albumins, to protect the peptides and proteins from degradation, aggregation or from loss of activity. The pharmaceutical compositions can be applied to an individual in need once or repeatedly. It can be applied one or more times a day, weekly, monthly or in longer time intervals. The pharmaceutical compositions can be used alone or in combination with other pharmaceutical compositions. Combined pharmaceutical compositions can have less than additive, can have additive and can have synergistic effects on the individual treated or tested. The pharmaceutical composition can comprise up to 3 µg, up to 10 µg, up to 30 µg, up to 100 µg, up to 300 µg, up to 1 mg, up to 30 mg, up to 300 mg, up to 1 gram of a TIF1-beta peptide and/or protein, nucleic acid coding for said peptide and/or protein, binding agent specifically binding to said peptide and/or protein, antibody specifically binding to said peptide and/or protein or of a fragment or derivate of the said or combinations thereof.

If the pharmaceutical composition is used as a diagnostic composition it can be used alone or in combination with other diagnostic compositions or diagnostic methods. The result of the diagnosis by combination of two or more pharmaceutical compositions and/or diagnostic methods can improve the reliability of the diagnosis less than additively or it can improve it additively or it can improve the diagnosis synergistically. The pharmaceutical composition can be used for diagnosis to prove the presence or to prove the absence of a disorder, preferably chosen from disorders caused by cells with a hyperproliferative disorder and/or a differentiation disorder and/or a neoplastic disorder, preferably chosen from cancer or from colorectal cancerous or related disorders..

### Suitable methods to analyze the sample

Generally all methods suitable to detect and analyze the peptides and/or proteins present in the sample can be used in the methods of the invention and can be used to determine the peptides and/or proteins and/or nucleic acids, preferably TIF1-beta and/or TIF1-beta fragments and/or nucleic acids coding for the said or the corresponding complementary nucleic acids or the corresponding peptides, proteins and nucleic acids listed in table 1. Preferably mass spectrometric methods, protein chip assays, immunology and molecular biology methods can be used.

With determining of peptides, proteins, TIF1-beta, TIF1-beta fragments or derivatives or polymorphic forms thereof is meant the qualitative or quantitative measurement of these substances. Depending on the sensitivity of the method used, the determination may have as a result that a substance is not present in a sample (e.g. it is present below the detection limit of the method used). If the quantity is determined the result may be a relative or an absolute value, depending on the method used.

Suitable mass spectrometric methods among others are matrix assisted laser desorption ionisation (MALDI), continuous or pulsed electrospray ionization (ESI) and related methods such as ionspray or thermospray or massive cluster impact (MCI). The ion sources can be matched with detection formats including linear or non-linear reflection time-of-flight (TOF), single or multiple quadrupole, single or multiple magnetic sector, fourier transform ion cyclotron resonance (FTICR), ion trap, and combinations thereof, e.g. ion-trap/time-of-flight. For ionization, numerous matrix/wavelength combinations (MALDI) or solvent combinations (ESI) can be used. Other mass spectrometric methods suitable are for example fast atom bombardment (FAB) mass spectrometry, Surface Enhanced Laser Desorption/lonization (SELDI) mass spectrometry, isotope coded affinity tag (ICAT) mass spectrometry, iTRAQ mass spectrometry (Applied Biosystems, Foster City, CA, USA) or affinity mass spectrometric methods.

Furthermore suitable immunologic methods among others are enzyme linked immuno assays (ELISA), sandwich, direct, indirect, or competitive ELISA assays, enzyme-linked immunospot assays (ELISPOT), radio immuno assays (RIA), flow cytometry assays (FACS = fluorescence activated cell sorting), immunohistochemistry, western blot, fluorescence resonance energy transfer (FRET) assays, protein-chip assays using for example antibodies, antibody fragments, receptors, ligands, or other agents binding the peptides and/or proteins of the invention, preferably binding agents specific for TIF1-beta fragments and/or TIF1-beta proteins.

Among others Northern or Southern blot hybridization, nucleic acid dot- or slot-blot hybridization, in situ hybridization, nucleic acid chip assays (for example using RNA, DNA or other nucleic acids to specifically capture the nucleic acids of interest), polymerase chain reaction (PCR), reverse transcriptase PCR (RT-PCR), real time PCR (taq-man PCR) among others can be used as molecular biology methods to detect the nucleic acids of the invention.

Further methods such as nuclear magnetic resonance (NMR), fluorometry, colorimetry, radiometry, luminometry, or other spectrometric methods, liquid chromatography, capillary chromatography, thin-layer chromatography, plasmon resonance (BIACORE), one- or two-dimensional gel electrophoresis, etc. can be used to detect the peptides and/or proteins and/or nucleic acids, preferably those corresponding to the sequences listed in table 1, preferably corresponding to TIF1-beta, preferably to human TIF1-beta.

In the following a protocol for the preparation of plasma samples is exemplied. Said protocol represents an alternative method to the method described in example 1 for obtaining plasma samples.
If not mice or rats but larger experimental animals or human patients are used to obtain blood, the blood preferably can be collected from the cubital vein into blood collection tubes. Alternatively other blood vessels of suitable size can be used. Preferably EDTA- or citrate-plasma, most preferably EDTA-plasma is used as sample. EDTA-plasma for example can be obtained by use of 9 ml S-Monovettes containing approximately 1.5 mg potassium-EDTA. Citrate-plasma for example can be obtained by 9 ml S-Monovettes containing 1 ml 0.106 mol/l citrate solution. In general, all kinds of anticoagulants in "anticoagulant-effective" concentrations can be used, as long as they do not interfere with chromatography, preferably do not interfere with reverse-phase chromatography, and do not interfere with subsequent peptide analysis by mass spectrometry and do not interfere with other steps of sample preparation, handling or analysis. Commonly used additional substances to stabilize a peptide or protein-containing sample such as protease inhibitors which often are proteins or peptides are not suitable, as they may mask the peptides of the sample. Anticoagulants such as heparin are not suitable as heparin is usually a mixture of heparin molecules of various molecular weights (about 6 000 to 40 000 Da) and heparin molecules have multiple negative charges. Therefore heparin results in for example numerous mass signals in mass spectrometry and may also result in difficulties with other methods such as ultrafiltration, size exclusion or ion exchange chromatography. Protease inhibitors, which are not peptides or proteins, may be compatible with the sample preparation and analysis described here. Preferably immediately after withdrawal, plasma can be obtained by centrifugation of the blood for 10 min. at 2000x g at room temperature. After withdrawal the blood should be stored at room temperature and not be cooled. Preferably the storage time after??? withdrawal should be below 1 hour, preferably below 30 min., preferably below 20 min., preferably below 10 min., most preferably below 5 min. The temperature of the Blood sample and the plasma or serum sample should be kept at a temperature between 37 and 10 °C, preferably between 30 and 12 C°, preferably between 26 and 15 °C, most preferably between 24 and 18 °C. Centrifugation should be done at room temperature or temperatures around room temperature but not at temperatures above 37°C or below 10°C. This improves the plasma quality, as cooling or heating of the blood sample can result in activation of thrombocytes which react by releasing peptides or proteins such as proteases and other enzymes into the plasma. These proteins, peptides, proteases and other enzymes bear the risk of potentially masking, covering, digesting or modifying in other ways marker peptides present in the plasma sample. After centrifugation residual thrombocytes can be removed from the plasma sample by filtering the plasma using for example a 10 ml syringe with attached filter, preferably a cellulose acetate filter unit with a pore size small enough to exclude thrombocytes from the filtrate, preferably with a pore size between 0.2 and 0.01 µm, preferably between 0.2 and 0.05 µm, preferably between 0.2 and 0.1 µm, most preferably with a pore size of 0.2 µm and a filtration area sufficient large enough to conveniently perform filtration, for example with a 5 cm² filtration area (Sartorius Minisart®, Sarstedt, Nümbrecht, Germany).

Another alternative protocol for plasma sample quality improvement without a filtration step is a two-step centrifugation-scheme. In the first step the blood is centrifuged gently for 5 to 30 min. at 800 to 2500x g, preferably for 10 min. at 2000x g, to avoid erythrocyte lysis. In the second step the plasma is transferred to a fresh tube and centrifuged more stringent to remove as many thrombocytes as possible without lysis of thrombocytes by centrifugation for 5 to 60 min. at 1000 to 3000x g, preferably for 15 min. at 2500x g. Both centrifugation steps have to be performed at temperatures around room temperature and not at temperatures above 37°C or below 10°C.

A protocol for preparation of plasma samples which is different to the protocol described in example 2 is described below. This protocol is also suitable for obtaining samples for subsequent analysis according to the present invention.

Thus, an alternative protocol for reduction of the protein load of plasma from mice, rats, humans and other species is a 1:3 dilution of the plasma sample with 8 M Guanidinium chloride solution at 23 °C and subsequent ultrafiltration using for example Amicon Ultra filter devices with a molecular weight cut of 150 to 10 kDa (Millipore, Bedford, USA), which are centrifuged according to the manufacturers instruction, preferably using Amicon Ultra filter devices with a molecular weight cut of 50 kDa centrifuged at 4000x g for 60 min. in a swinging bucket rotor.

The following protocol for preparation of samples from in vitro cultured cells represents an approach to prepare samples from in vitro cultured cells. Another approach is described in Example 3.

Sample preparation can be done by removing the tissue culture medium from the cell culture plates, adding extraction buffer (6M Guanidinium-chloride, 50 mM Glycine, pH adjusted to 2.6 by use of HCI) to the cell layer and scraping off the cells from tissue culture plate using a rubber policeman. This step is repeated once and the resulting cell culture lysates are combined to one sample. For a 7 cm diameter tissue culture plate 1 ml of extraction buffer is used for each of these two steps. The cell extracts are heated to 99 °C for 5 min. in a thermo mixer shaking at 1100 rpm. After centrifugation at 18 000x g for 15 min. the supernatant is harvested and subjected to chromatography or stored at -80°C until further used. All extraction steps including the centrifugation are performed at room temperature.

The following protocol for preparation of tissue samples represents an alternative to the protocol described in example 5.

Sample preparation from tumor tissue can be done by homogenization of 20 to 50 mg tumor tissue in the presence of 1 ml extraction buffer (6M Guanidinium-hydrochloride, 50 mM Glycine, pH adjusted to 2.6 by use of HCI). Subsequently the tumor extracts are heated to 99 °C for 5 min. in a thermo mixer shaking at 1100 rpm. After centrifugation at 18 000x g for 15 min. the supernatant is harvested and subjected to chromatography or stored at -80°C until further used. Homogenization of the tumor tissue is done at low temperatures below 0°C and all subsequent extraction steps including the centrifugation are performed at room temperature.

### Test kits

A further aspect of the invention are test kits for determining the absolute concentration or the relative concentration or the presence or absence of a peptide and/or protein and/or nucleic acid, preferably of a TIF1-beta peptide and/or protein and/or nucleic acid or a fragment or derivative or polymorphic form thereof. The test kit can be used to diagnose the absence or presence of disorders caused by cells with a hyperproliferative disorder, and/or a differentiation disorder and/or a neoplastic disorder, preferably cancer cells or a colorectal cancerous cells or cells with related disorders. The test kit further can be used to predict the occurrence or to predict the grade of said disorder and/or to predict and/or monitor the success of a therapy for said disorder and/or to predict and/or to monitor a relapse of a said disorder. Preferably the test kit can be used to early diagnose and/or predict said disorder, preferably before the diagnosis is possible by other diagnostic tests which may be known in the art. The test kit can be used to analyze any kind of sample from an individual including but not limited to whole blood, blood cells (such as leukocytes, B-cells, T-cells, monocytes, macrophages, erythrocytes and thrombocytes), serum, plasma, hemofiltrate, liquor cerebrospinales, urine, lymph, lymph knot tissue, stool, tissue from the colon or the rectum, cancer tissue, tumors, tissue of polyps or other pre-cancerous tissues or other tissues and organs as well as combinations thereof. The test can be done with pooled samples of one or more than one individual. The sample can be used directly or can be a pre-processed, for example the sample can be fractionated by chromatography, filtration, precipitation, liquid or other extraction methods, immunoprecipitation, etc. The whole sample or individual or two or more combined fractions of a pre-processed sample, originating from one or more than one samples can be analyzed. The test can be done once or several times, preferably several times over a period of time to analyze the time course of quantity changes of a peptide or protein or nucleic acid, preferably of TIF1-beta, TIF1-beta fragments, or TIF1-beta nucleic acids. The test can be done with any type of test kit known in the art including but not limited to the methods to analyze a sample as described elsewhere in this patent application. The test kit can comprise substances such as TIF1-beta, TIF1-beta fragments, TIF1-beta nucleic acids and/or fragments and/or derivatives and/or polymorphic forms of the said for use as standard and/or for use as a control. The kit can comprise agents binding specifically to said TIF1-beta and/or TIF1-beta fragments or hybridizing specifically to the corresponding nucleic acids. Furthermore the test kit of the invention can comprise instructions how to use the test kit, how to prepare the samples, what kind of samples to use, how to analyze and interpret the results, etc. Especially the test kit can comprise instructions, how to use the kit for determining the presence or absence or prognosis of cells with a hyperproliferative disorder and/or of cells with a differentiation disorder and/or of neoplastic cells, preferably how to use the test kit for diagnosis or prognosis of the presence of cancer cells or pre-cancerous cells, preferably originating from colorectal cancerous or related disorders.

### Identified markers

In the invention several markers were detected and/or identified using neoplastic cells namely HCT-116 cells, grown in immune-compromised non-human hosts, namely SCID mice, by analysis of plasma samples of the experimental animals. These markers were fragments of certain proteins, namely of TIF1-beta, pro-Thymosin alpha (PTA) and cytokeratin 18 (CK18). The exact sequences, sequence positions within the corresponding complete protein sequences and the corresponding Seq.-IDs of the sequence protocol are depicted in table 1. In addition some further peptides were identified as markers of neoplastic cells and/or as markers of cells with a hyperproliferative disorder and/or as markers of cells with a differentiation disorder using the methods described herein. The masses of these peptides were 2315 Da and 2916 Da, but the amino acid sequences of these peptides were not determined. Both peptides were identified in fraction 35. Table 1 shows, in which of the different kind of samples the peptides of the invention were found.

The fragments of these proteins are unknown in the prior art and were not known to be suitable as marker for cells with a hyperproliferative disorder and/or cells with a differentiation disorder and/or neoplastic cells, preferably of cancer cells or colorectal cancerous cells or of cell with related disorders. Especially it was unknown in the prior art, that TIF1-beta or fragments or derivatives or polymorphic forms thereof are released into the organism of a host from cells growing in a host, wherein said cells are cells with a hyperproliferative disorder and/or cells with a differentiation disorder and/or neoplastic cells. TIF1-beta (Transcription intermediary factor 1-beta ) is a transcription factor and therefore expected to be present in the nucleus of the cell. Therefore it was surprising to find extracellular TIF1-beta or fragments or derivatives thereof as markers for the presence of cells with a hyperproliferative disorder and/or cells with a differentiation disorder and/or neoplastic cells in the organism of the host of said cells.

**Table 1: Peptides identified by MALDI to be originating from the xenograft (HCT-115 colon tumor cells) and types of samples comprising said peptides**

| Mass detected [m/z] ⁽¹⁾ | 3169 | 3788/3845 | 2315 | 2916 | 2422 |
|---|---|---|---|---|---|
| Identity | TIF1-beta | PTA⁽²⁾ | unknown | unknown | CK18 |
| Sequence position | 2-39 ⁽³⁾ | 2-36/2-37 | unknown | unknown | 410-430 |
| Seq.-ID. | 1 | 9/10 | --- | --- | 11 |
| Correlation r of tumor size with MALDI mass signal intensity | 0.97 | 0.96/ --- | 0.85 | 0.78 | 0.50 |
| SCID-mice plasma (tumor positive) | Yes | Yes / Yes | Yes | Yes | Yes |
| SCID-mice plasma (tumor negative) | No | (yes)⁽⁴⁾ / No | No | No | No |
| SCID-mice tumor tissue | Yes | Yes / Yes | Yes | No | Yes |
| in vitro cultured tumor cells | Yes | Yes / Yes | Yes | No | Yes |
| supernatant of in vitro cultured tumor cells | Yes | Yes / Yes | No | No | Yes |
| plasma pool of colon carcinoma patients | Yes⁽⁵⁾ | --/-- | Yes | Yes | No |

| | | | | | |
|---|---|---|---|---|---|
| ⁽¹⁾ m/z = mass / charge of the ion, TIF1-b = transcription intermediary factor-1 beta, PTA = pro-Thymosin alpha, CK18 = cytokeratin 18. Indicated masses are theoretic monoisotopic masses. Experimentally determined masses vary slightly from these masses due to naturally occurring different isotopes of elements with slightly different masses. | | | | | |
| ⁽²⁾ Peptides originating form prothymosin alpha (PTA) were also found in this study and are documented in a separate patent application (unpublished patent application No. EP 04029171.8) | | | | | |
| ⁽³⁾ All amino acid counts start with the N-terminal methionine. The sequences of TIF1-beta (2-39) is: AASAAAASAAAASAASGSPGPGEGSAGGEKRSTAPSAA (Seq.-ID 1), the sequence of PTA (2-36) is SDAAVDTSSEITTKDLKEKKEWEEAENGRDAPAN (Seq.-ID 9), the sequence of PTA (2-37) is SDAAVDTSSEITTKDLKEKKEWEEAENGRDAPANG (Seq.-ID 10) and the sequence of CK18 (410-430) is TRRIVDGKWSETNDTKVLRH (Seq.-ID 11). | | | | | |
| ⁽⁴⁾ MALDI mass signal intensity in tumor negative SCID-mice plasma is clearly lower, as compared to tumor-positive SCID-mice | | | | | |
| ⁽⁵⁾ Only detected after second chromatographic separation step | | | | | |

### EXAMPLES

The following examples are intended to illustrate the invention, however they are not meant to limit the scope of the invention in any way.

### Example 1: Growth of tumor cells, injection into mice and sample collection

The human colorectal carcinoma cell line HCT-116 was obtained from ATCC, Manassas, VA, USA, order no. CCL-247. The tumor cells were cultured in RPMI 1640 medium supplemented with 10 % fetal bovine serum and 2 mM L-glutamine at 37 °C with 5 % CO₂. All tissue culture media and supplements were from Gibco BRL, Gaithersburg, MD, USA. Cell passaging of the adherent tumor cells was performed by splitting the cell culture twice a week at a ratio 1:10. At the day of injection (time point zero) cells were harvested from the culture flasks using Trypsin /ethylenediaminetetraacetic acid (EDTA) solution as known in the art and transferred into culture medium, washed once and resuspended in phosphate buffered saline (PBS). After an additional washing step with PBS the final cell concentration was adjusted to 15 000 000 live cells per ml. The tumor cell suspension was carefully mixed to avoid cell aggregation, kept on ice and drawn into a 1.0 ml syringe without attached needle. 200 µl cell suspension (containing 3 000 000 cells) were subcutaneously injected into the right lateral flank of the mice using a needle with a size of 0.45 x 25 mm. A total of 60 mice were injected with tumor cells. 40 additional animals were used to obtain negative control samples and were injected with PBS only. The animals with a tumor were sacrificed on days 7, 13 or 34 after tumor cell injection. Control groups of 20 animals each were sacrificed on days 7 and 34. EDTA-blood was obtained by cardiac puncture at the time points indicated. EDTA plasma was prepared as known in the art and samples from individual animals were stored at -80 °C within 30 minutes after blood drawing. Weights and volume of the explanted primary HCT-116-tumors were determined at the time point of dissection. The tumor volume was calculated by multiplying the length and the squared average width measured in centimeter resulting in milliliter (ml) of tumor volume. Immediately after weighing and determining the volume the individual tumors were frozen in liquid nitrogen and stored at -80 °C until further processed.

### Example 2: Sample preparation using murine plasma

Prior to analysis a reduction of the protein load of the plasma samples was achieved by trichloroacetic acid (TCA) precipitation as known in the art. Samples of 0.5 ml plasma were added to 1 ml chilled, distilled water. For protein precipitation 0.5 ml TCA (20 % (v/v) concentration of TCA) were added and the tubes vigorously mixed for 30 seconds. After incubating for 30 minutes on ice, samples were centrifuged at 18 000 g for 30 minutes at 4 °C. The supernatants were transferred into new tubes and stored at -80 °C until further processed. A 375 µl-equivalent of plasma was used per chromatographic run.

### Example 3: Sample preparation using in vitro cultured cells

Cells were removed from tissue culture plates by use of trypsin/EDTA and a rubber policeman, centrifuged for 5 minutes at 600 g at room temperature and washed twice with PBS. For peptide extraction moist mass standardized samples of 50 000 to 400 000 cells were used. After addition 200 µl of 200 mM acetic acid the samples were boiled for 10 minutes. Subsequently the pH of the samples was adjusted to pH 2 to pH 3 using a 30 % stock solution of HCI and finally the samples were diluted to a final volume of 1.5 ml using a 0.1 % stock solution of TFA. After 10 minutes centrifugation at 18 000 g at 4 °C the supernatant was harvested and stored in tubes at -80 °C until further processed. Cell extracts, corresponding to 50 000 to 400 000 cells were used per chromatographic run.

### Example 4: Sample preparation using cell culture supernatants

Using a 10 ml syringe the cell culture supernatant was passed through a cellulose acetate filter with 0.2 µm pore size and 5 cm² filtration area (Minisart®, Sartorius AG, Göttingen, Germany). The pH of the filtered sample was adjusted to pH 2 to pH 3 using a 30 % (v/v) stock solution of HCl and stored at -80 °C until further processed. 1.5 mL of cell culture supernatant was used per chromatographic run.

### Example 5: Sample preparation using HCT-116 tumors, isolated from SCID mice

The mass of the samples was determined by weighing. After addition of 80 µL of 200 mM acetic acid to 4 mg moist mass of tumor tissue, samples were boiled for 10 minutes. Typically 20 to 50 mg tumor tissue were used as staring material. Subsequently the pH of the sample was adjusted to pH 2 to pH 3 using a 30 % stock solution of HCI and finally the sample was diluted to 1.5 ml using a 0.1 % stock solution of TFA. After 10 minutes centrifugation at 18 OOOx g at 4 °C the supernatant was harvested and stored at -80 °C until further processed. Cell extracts, corresponding to 4 mg tumor tissue were used per chromatographic run.

### Example 6: Liquid chromatography of the samples

The separation method carried out was reverse phase chromatography. Various RP chromatography resins and buffers are equally suitable. The separation of peptides and/or proteins using a C18 reverse phase chromatography column having a size of about 4 mm x 250 mm was done as described below. Here, a Source RPC, 4,6 x 150 mm (Amersham Biosciences Europe GmbH, Freiburg, Germany) is used. Buffer A was 0.06 % volume per volume (v/v) trifluoroacetic acid (TFA) and 99,94 % (v/v) distilled water and buffer B was 0.05 % (v/v) TFA, 80 % (v/v) acetonitrile and 19.95 % (v/v) distilled water. The chromatography took place at 33 °C using a HP 1100 HPLC with a micro flow cell both supplied by Agilent Technologies, Böblingen, Germany. The pH of the sample was adjusted to pH 2 to pH 3 using a stock solution of 30 % (v/v) HCI and subsequently the samples were diluted to 1.5 ml using a 0.05 % (v/v) stock solution of trifluoroacetic acid. Prior to chromatography the samples were centrifuged at 4 °C and 18 000 g for 10 minutes and finally 1.5 ml of sample were loaded onto the chromatography column representing an equivalent of 375 µl plasma, 50 000 to 400 000 in vitro cultured cells, 4 mg moist weight of tumor tissue or 1.5 ml cell culture supernatant. Buffers A and B were mixed in various ratios during chromatography and subsequently only the percentage of buffer B is stated. The percentage of buffer A is 100 % minus % buffer B (v/v). The chromatography conditions were as follows:
- 5 % buffer B for 1 min,
- a linear gradient from 5 to 50 % buffer B during the next 44 min.
- a linear gradient from 50 to 100 % buffer B during the next 4 min.
- 100 % buffer B for the next 4 min.

The flow rate was 500 µl/min. and 96 fractions, each containing 0.25 mLl were collected during the gradients from 5 to 100 % buffer B.

### Example 7: Collection and preparation of human plasma

Plasma samples from 19 male colon carcinoma patients of various cancer stages, having a mean age of 62 years were collected by standard methods known in the art using EDTA as anti-coagulant. The collection tubes were centrifuged for 10 min. at 2000 g and 4 °C. Plasma samples were stored at -80 °C until further processed. Prior to chromatography 4 ml of a pool of these 19 plasma samples were subjected to trichloroacetic acid (TCA) precipitation as known in the art. Briefly, aliquots of 500 µl plasma were added to 1 ml chilled, distilled water. Protein precipitation was carried out by adding 500 µl of ice cold 20 % (v/v) TCA and vigorously mixing of the tubes for 30 seconds. After incubating for 30 min. on ice, the tubes were centrifuged for 30 minutes at 18 000 g and 4 °C. The supernatants were used for further analysis. TCA precipitation was done to remove high molecular weight proteins and to enrich peptides and low molecular weight proteins. Subsequently the supernatant of the TCA precipitation was separated into 96 fractions as described in example 6. A second fractionation using a 3.6 ml plasma equivalent of fraction 42 of the first fractionation (according to example 6) was done using a Jupiter C18 reverse phase column with a size of 2 x 250 mm (Phenomenex, Aschaffenburg, Germany). The chromatography took place at 33 °C using a HP 1100 HPLC with a micro flow cell both supplied by Agilent Technologies, Boblingen , Germany. Buffer A was 0.06 % (v/v) trifluoroacetic acid (TFA) and 99.94 % (v/v) distilled water and buffer B was 0.05 % (v/v) TFA, 80 % (v/v) acetonitrile and 19.95 % (v/v) distilled water. The chromatographic conditions were as follows:
- 5 % buffer B during the first minute
- a linear gradient from 5 to 15 % of buffer B during the next 2 minutes
- a linear gradient from 15 to 40 % of buffer B during the next 42 minutes
- a linear gradient from 40 to 100 % buffer B for the next 4.minutes
- 100 % buffer B during the next 4 minutes
- a linear gradient from 100 to 50 % buffer B during the next 1.5 minutes
- a linear gradient from 50 to 100 % buffer B during the next 3 minutes and
- finally a linear gradient from 100 to 50 % buffer B during the final 2.5 minutes.

The flow rate was 100 µl/min. and 96 fractions, each containing 50 µl were collected during the gradients from 5 to 100 % buffer B.

### Example 8: Mass spectrometry of the samples

For mass spectrometric analysis, typical positive ion spectra of peptides were measured using a MALDI-TOF (matrix-assisted laser desorption ionization time of flight) mass spectrometer. Suitable MALDI-TOF mass spectrometers among others are manufactured by PerSeptive Biosystems, Framingham, USA (Voyager-DE, Voyager-DE PRO or Voyager-DE STR) or by Bruker Daltonik, Bremen, Germany (BIFLEX). Typical matrix substances suitable for peptides are matrix substances which comprise an organic acid such as 3,5-dimethoxy-4-hydroxycinnamic acid, alpha-cyano-4-hydroxycinnamic acid and 2,5-dihydroxybenzoic acid. For MALDI sample preparation we used alpha-cyano-4-hydroxycinnamic acid as matrix and 6-desoxy-I-galactose as co-matrix, dissolved in acetonitrile containing 0.1 % TFA. A lyophilized equivalent obtained by reverse phase chromatography corresponding to 7.5 µl plasma or 1000 to 5000 of in vitro cultured cells or 80 µg of tumor tissue or 30 µl cell culture supernatant spotted onto the mass spectrometric carrier plate was used to measure the peptides and/or proteins. The fractionated, lyophilized sample was dissolved in 15 µl of a matrix solution. This matrix solution contained, for example, 10 g/L α-cyano-4-hydroxycinnamic acid and 10 g/L L(-)fucose dissolved in a solvent mixture consisting of acetonitrile, water, trifluoroacetic acid (TFA) and acetone in a ratio of 49:49:1:1 by volume (v/v). 0.3 µl of this sample solution was transferred to a MALDI carrier plate, and the dried sample was analyzed in a Voyager-DE STR MALDI mass spectrometer from PerSeptive Biosystems. The measurement took place in linear mode with delayed extraction™. A MALDI-TOF mass spectrometer can be employed to quantify peptides such as, for example, the peptides of the invention if these peptides are present in a concentration which is within the dynamic measurement range of the mass spectrometer, thus avoiding detector saturation. The ability of MALDI mass spectrometry to quantitate individual peptide and/or protein mass signals was confirmed by adding known concentrations of 50 to 800 pmol peptides, such as neurotensin, into complex biological samples such as plasma samples. Subsequently these samples were separated by reversed phase chromatography and those fractions containing the added peptides were analyzed by mass spectrometry as described in the examples. There is a the linear correlation between the MALDI mass spectrometry mass signal intensity of for example the neurotensin peptide and the amount of the neurotensin peptide added to the plasma sample, indicating that MALDI mass spectrometry is suitable to quantitatively determine peptides in complex samples such as plasma (figure 2).

### Example 9: Peptide identification

Detection of differentially expressed peptides was achieved by calculation of subtractive peptide display maps and correlation analysis. Selected peptides, which appeared only in peptide display maps of plasma from mice with a tumor, cell culture or tissue specimens but not in control mice plasma were analyzed by ESI-qTOF sequencing (PE Applied Biosystems, Framingham, USA). Peptide ions were selected in the mass spectrometer on the basis of their specific m/z (mass/charge) values in a manner known to the skilled worker. These selected ions were then fragmented by supplying collision energy (20 - 40 eV) with an collision gas, e.g. helium or nitrogen, and the resulting fragments of the peptides or proteins were detected in the mass spectrometer in an integrated analysis unit, and corresponding m/z values were determined (principle of tandem mass spectrometry). The fragmentation behavior of peptides enables unambiguous identification of the peptides. The mass-spectrometric analysis for example can be done for example by use of a Quadrupol-TOF (time of flight) sequencing (QStar-Pulsar model) or by ESI-qTOF sequencing (both from PE Applied Biosystems, Framingham, USA). The resulting peptide fragment spectra were detected using nanoSpray in the product ion scan mode (spray voltage 950 V, collision energy 20-40 eV). Up to 200 scans per sample were accumulated. Charge state deconvolution was performed using the Bayesian reconstruct tool of the BioAnalyst program package (PE Applied Biosystems), deisotoping was achieved by means of the Voyager 5.1 software (PE Applied Biosystems). The mass spectra were saved in a MASCOT generic file format, and submitted to the MASCOT19 database search engine (Matrix Science, London, UK). Searched databases were SwissProt (Version 39.6, www.expasy.ch) and MSDB (Version 010721, EBI, Europe). In addition, this peptide sequencing method allows the identification of amino acid modifications such as phosphorylation or acetylation that are often involved in the regulation of the biological activity of proteins and peptides.

MALDI-PSD-MS measurements of single HPLC-fractions of tissue specimen were also performed using a Voyager ToF mass spectrometer (Applied Biosystems, Framingham, MA, USA).

### Example 10: Data analysis

Subsequently to fractionation as described in example 6 or 7, each fraction was individually analyzed by MALDI mass spectrometry as described in example 8 resulting in 96 mass spectra for each sample. These 96 mass spectra are electronically combined to a so called peptide display. The x-axis of these peptide displays depicts the molecular mass, the y-axis depicts the fraction number and the color intensity (grey scale) represents the mass spectrometric signal intensity. The data of peptide displays may be pre-processed by adjusting for background noise and outliers may be removed from the analysis. Differences between peptide displays are calculated by subtracting peptide displays from each other electronically and/or by correlation analysis of mass signal intensities. For correlation analysis every mass signal intensity above a threshold mass signal intensity was tested for correlation to any other mass signal intensity above the same certain threshold of mass signal intensity. Detection of different (relative) concentrations of peptides and/or proteins was done by comparison of the mass spectrometric data (mass signal intensities) from samples to be compared. Peptide displays from cell extracts from in vitro cultured tumor cell or from in vivo in SCID mice grown tumor cells or from tissue culture supernatants or from serum of SCID mice or from serum of human colon cancer patients gave about 1000 to 2300 non-redundant peptide and/or protein mass signals.

Various peptide and/or protein mass signals were identified in plasma of SCID mice which clearly originated from the xenograft (the implanted, human HCT-116 colon tumor cell line). These peptides and/or proteins were detected in the plasma of the xenograft-transplanted SCID MICE but not in the control mice injected with PBS instead of tumor cells (xenograft). In addition some of these various peptides were also identified in cell culture supernatants of in vitro cultured HCT-116 colon tumor cells, in cell extracts of in vitro cultured HCT-116 colon tumor cells and/or in cell extracts of tumors comprising HCT-116 cells which tumors were isolated from SCID mice. Table 1 summarizes the peptides detected and/or identified and in which kind of samples they were detected. Peptides whose sequences were identified are indicated by the respective sequence position within the complete sequence of the mother molecule/protein from which they originate.

## Claims

1. A method for the detection of the presence or absence of cells in an individual, wherein said cells are chosen from the group consisting of cells having a hyperproliferative disorder, cells having a differentiation disorder and neoplastic cells comprising the steps of:
a) determining the amount of at least one TIF1-beta and/or at least one TIF1-beta fragment and/or a derivative and/or polymorphic form thereof in a sample of said individual;
b) comparing the result of a) with the result determined using a negative control sample or with a known reference value; and
c) determining the presence or absence of said cells in said individual based on the result of the comparison made in step b).

2. The method according to claim 1, wherein the at least one TIF1-beta fragment is selected from the group consisting of peptides according to Seq.-ID 1 to 5 and/or derivatives and/or polymorphic forms thereof.

3. The method according to any one of the preceding claims for determining in said individual the presence of cancer cells or of cells having a colorectal cancerous or a related disorder.

4. The method according to any one of the preceding claims, wherein said sample is selected from the group consisting of whole blood, plasma, serum, cerebrospinal fluid, lymph, urine, stool and an tissue sample.

5. The method according to any one of the preceding claims, wherein said TIF1-beta and/or said fragment of TIF1-beta and/or a derivative and/or polymorphic form thereof is determined by a method selected form the group consisting of ELISA, RIA, western blot, protein chip assays, mass spectrometry, immune histology, flow cytometry or by molecular biologic methods.

6. A peptide and/or a derivative and/or a polymorphic form thereof comprising a fragment of TIF1-beta, which fragment is absent or present in altered quantities in an individual, wherein said individual comprises cells chosen from the group consisting of cells having a hyperproliferative disorder, cells having a differentiation disorder and neoplastic cells as compared to the quantities of said peptide in an individual not comprising said cells, with the proviso that said peptide is not chosen from the group consisting of Seq.-ID 6 or 7.

7. The peptide according to claim 6, wherein said peptide is present in an individual comprising cancer cells, or comprising cells having a colorectal cancerous or a related disorder.

8. A method to identify TIF1-beta fragments and/or derivatives and/or polymorphic forms thereof, which TIF1-beta fragments are present in a sample of the organism of a host comprising a xenograft, wherein said xenograft releases said TIF1-beta fragments or wherein said xenograft releases precursors of said TIF1-beta fragment which are subsequently modified, comprising the steps of
a) implanting a xenograft into the organism of a non-human host and letting grow said xenograft for a certain amount of time within said organism of said non-human host,
b) collecting a sample from said organism of a non-human host,
c) determining the presence, absence or quantity of a plurality of peptides present in said sample,
d) identifying those peptides determined in step c) which are absent or which are present in altered quantities in a sample of the organism of a non-human host not transplanted with said xenograft and which peptides are TIF1-beta fragments and/or a derivatives and/or polymorphic forms thereof.

9. TIF1-beta fragments and/or derivatives and/or polymorphic forms thereof identified with the method according to claim 8, with the proviso that the peptides according to Seq.-ID 6 and 7 are not included.

10. A peptide according to any one of claims 6, 7 or 9 which has a sequence according to any one of Seq.-IDs 1 to 5.

11. The peptide according to any one of claims 6, 7, 9 or 10 wherein the peptide comprises at least one additional non-TIF1-beta amino acid sequences.

12. Binding agents specifically binding to a neoepitope of a peptide according to Seq.-ID 1 to 5, which binding agents do not bind a peptide according to Seq.-ID6or7.

13. Binding agents according to claim 12 which are antibodies, fragments or derivatives thereof.

14. Nucleic acids selected from the group consisting of
a) a nucleic acid sequence encoding a peptide according to any one of claim 6, 7 or 9 to 11;
b) a nucleic acid sequence which is complementary to the nucleic acid sequence under a);
c) a nucleic acid sequence, which hybridizes under stringent conditions to a nucleic acid under a) and/or b) and which at maximum has a nucleic acid sequence length which is shorter than the nucleic acid sequence coding for Seq.-ID 6 or 7;
d) a nucleic acid sequence, which is degenerated with respect to said nucleic acid sequence under any one of a) to c);
e) a derivative and/or fragment of a nucleic acid sequence according to any one of a) to d);
f) a nucleic acid sequence according to a), b), c), d) or e) further comprising at least one non-TIF1-beta nucleic acid sequence; and/or
g) a nucleic acid sequence according to f), wherein said at least one non-TIF1-beta nucleic acid sequence is a sequence for labelling of the nucleic acid sequence.

15. The nucleic acid according to claim 14 further comprising a label, which label is not a nucleic acid sequence.

16. A vector comprising a nucleic acid according to claim 14 or 15.

17. A host cell comprising a nucleic acid according to any one of claims 14 or 15 or a vector according to claim 16.

18. A test kit comprising
a) a peptide according to claim 6, 7, or 9 to 11; and/or
b) a binding agent according to claim 12 or 13; and/or
c) a nucleic acid according to claim 14 or 15; and/or
d) a vector according to claim 16; and/or
e) a host cell according to claim 17, and
instructions how to use the test kit for determining the presence, absence, prognosis or relapse of a disorder.

19. A test kit comprising
a) a peptide according to claim 6, 7, or 9 to 11; and/or
b) a peptide which is TIF1-beta or a fragment and/or a derivative and/or a polymorphic form thereof; and/or
c) a peptide according to b) which in addition comprises at least one non-TIF1-beta amino acid; and/or
d) a binding agent according to claim 12 or 13; and/or
e) a binding agent specific for TIF1-beta or specific for a fragment or derivative or polymorphic form thereof; and/or
f) a nucleic acid according to claim 14 or 15; and/or
g) a nucleic acid which is coding for TIF1-beta and/or a fragment and/or derivative and/or a polymorphic form thereof; and/or
h) a nucleic acid sequence which is complementary to or which hybridizes under stringent conditions to a nucleic acid according to f) or g); and/or
i) a nucleic acid according to f), g) or h) which in addition comprises at least one non-TIF1-beta nucleotide; and/or
j) a vector according to claim 16; and/or
k) a host cell according to claim 17, and
instructions how to use said test kit for a method according to any one of claims 1 to 5.

20. A pharmaceutical composition comprising
a) a peptide according to claim 6, 7, or 9 to 11; and/or
b) a binding agent according to claim 12 or 13; and/or
c) a nucleic acid according to claim 14 or 15; and/or
d) a vector according to claim 16; and/or
e) a host cell according to claim 17.

21. A pharmaceutical composition for treatment and/or prophylaxis and/or diagnosis of a disease selected from the group consisting of cancer, colorectal cancerous or related disorders, comprising
a) a peptide according to claim 6, 7, or 9 to 11; and/or
b) a peptide which is TIF1-beta or a fragment and/or a derivative and/or a polymorphic form thereof; and/or
c) a peptide according to b) which in addition comprises at least one non-TIF1-beta amino acid; and/or
d) a binding agent according to claim 12 or 13; and/or
e) a binding agent specific for TIF1-beta or specific for a fragment or derivative or polymorphic form thereof; and/or
f) a nucleic acid according to claim 14 or 15; and/or
g) a nucleic acid which is coding for TIF1-beta and/or a fragment and/or derivative and/or a polymorphic form thereof; and/or
h) a nucleic acid sequence which is complementary to or which hybridizes under stringent conditions to a nucleic acid according to f) or g); and/or
i) a nucleic acid according to f), g) or h) which in addition comprises at least one non-TIF1-beta nucleotide; and/or
j) a vector according to claim 16; and/or
k) a host cell according to claim 17, and

22. Use of
a) a peptide according to claim 6, 7, or 9 to 11; and/or
b) a binding agent according to claim 12 or 13; and/or
c) a nucleic acid according to claim 14 or 15; and/or
d) a vector according to claim 16; and/or
e) a host cell according to claim 17, and
for the manufacture of a test kit according to 18 or 19 and/or a pharmaceutical composition according to claim 20 or 21.

23. Use of
a) a peptide according to claim 6, 7, or 9 to 11; and/or
b) a peptide which is TIF1-beta or a fragment and/or a derivative and/or a polymorphic form thereof; and/or
c) a peptide according to b) which in addition comprises at least one non-TIF1-beta amino acid; and/or
d) a binding agent according to claim 12 or 13; and/or
e) a binding agent specific for TIF1-beta or specific for a fragment or derivative or polymorphic form thereof; and/or
f) a nucleic acid according to claim 14 or 15; and/or
g) a nucleic acid which is coding for TIF1-beta and/or a fragment and/or derivative and/or a polymorphic form thereof; and/or
h) a nucleic acid sequence which is complementary to or which hybridizes under stringent conditions to a nucleic acid according to f) or g); and/or
i) a nucleic acid according to f), g) or h) which in addition comprises at least one non-TIF1-beta nucleotide; and/or
j) a vector according to claim 16; and/or
k) a host cell according to claim 17, and
for the manufacture of a test kit and/or a pharmaceutical composition for treatment and/or prophylaxis and/or diagnosis of a disease selected from the group consisting of cancer, colorectal cancerous or related disorders.
